(19) Europäisches Patentamt
European Patent Office
Office européen des brevets

(11) EP 0 931 063 B3

(12) **NEUE EUROPÄISCHE PATENTSCHRIFT**
Nach dem Beschränkungsverfahren (B3-1)

(45) Veröffentlichungstag und Bekanntmachung des Hinweises auf die Entscheidung über den Antrag auf Beschränkung:
**B3-1 08.04.2009 Patentblatt 2009/15**

(45) Hinweis auf die Patenterteilung:
**18.10.2006 Patentblatt 2006/42**

(21) Anmeldenummer: **97937586.2**

(22) Anmeldetag: **16.08.1997**

(51) Int Cl.:
***C07D 209/18*** *(2006.01)* ***C07D 401/12*** *(2006.01)*
***A61K 31/40*** *(2006.01)*

(86) Internationale Anmeldenummer:
**PCT/EP1997/004474**

(87) Internationale Veröffentlichungsnummer:
**WO 1998/009946 (12.03.1998 Gazette 1998/10)**

(54) **N-SUBSTITUIERTE INDOL-3-GLYOXYLAMIDE MIT ANTIASTHMATISCHER, ANTIALLERGISCHER UND IMMUNSUPPRESSIVER/IMMUNMODULIERENDER WIRKUNG**

N-SUBSTITUTED INDOL-3-GLYOXYLAMID WITH ANTIASTHMATIC, ANTIALLERGIC AND IMMUNOSUPPRESSIVE/IMMUNOMODULATING EFFECT

INDOL-3-GLYOXYLAMIDES SUBSTITUES EN N AUX PROPRIETES ANTIASTHMATIQUES, ANTIALLERGIQUES ET IMMUNOSUPPRESSEURS/IMMUNOMODULATRICES

(84) Benannte Vertragsstaaten:
**AT BE CH DE DK ES FI FR GB GR IE IT LI LU MC NL PT SE**

(30) Priorität: **06.09.1996 DE 19636150**

(43) Veröffentlichungstag der Anmeldung:
**28.07.1999 Patentblatt 1999/30**

(73) Patentinhaber: **ZIOPHARM ONCOLOGY, INC.**
**New York, NY 10036 (US)**

(72) Erfinder:
- **LEBAUT, Guillaume**
  **F-44230 Saint-Sébastien-sur-Loire (FR)**
- **MENCIU, Cécilia**
  **F-44100 Nantes (FR)**
- **KUTSCHER, Bernhard**
  **D-63477 Maintal 1 (DE)**
- **EMIG, Peter**
  **D-63486 Bruchköbel (DE)**
- **SZELENYI, Stefan**
  **D-90571 Schwaig (DE)**
- **BRUNE, Kay**
  **D-91080 Marloffstein (DE)**

(74) Vertreter: **Müller-Boré & Partner**
**Patentanwälte**
**Grafinger Strasse 2**
**81671 München (DE)**

(56) Entgegenhaltungen:
**EP-A- 0 675 110** **DE-A- 1 595 924**
**FR-A- 2 689 888** **GB-A- 1 028 812**

EP 0 931 063 B3

## Beschreibung

Indol-3-glyoxylamide finden als pharmakodynamisch aktive Verbindungen und als Synthesebausteine in der pharmazeutischen Chemie eine vielfältige Verwendung.

In der Patentanmeldung NL 6502481 werden Verbindungen beschrieben, die über ein antiinflammatorisches und antipyretisches Wirkprofil und analgetische Aktivität verfügen.

In der britischen Anmeldung GB-PS 1 028 812 finden Derivate der Indolyl-3-glyoxylsäure und deren Amide Erwähnung als analgetisch, antikonsulsiv und β-adrenergisch wirksame Verbindungen.

G. Domschke et al. (Ber. 94, 2353 (1961)) beschreiben 3-Indolyl-glyoxylamide, die pharmakologisch nicht charakterisiert sind.

E. Walton et al. berichten in J. Med.Chem. 11, 1252 (1968) über Indolyl-3-glyoxylsäure-Derivate, die inhibitorisch auf die Glycerophosphat-Dehydrogenase und Lactat-Dehydrogenase wirken.

In der Europäischen Patentschrift EP 0 675 110 A1 werden 1 H-Indol-3-glyoxylsäureamide beschrieben, die als sPLA2-Inhibitoren profiliert werden und bei Behandlung des septischen Schocks, bei Pankreatitis, bei der Behandlung allergischer Rhinitis und rheumatischerArthritis zur Anwendung kommen. FR-A-2 689 888 beschreibt Perhydroisoindole, die eine spezifisch gerichtete, antagonistische Wirkung aufweisen. DE-A-1 595 924 beschreibt α-Hydroxy-, α-Alkoxy- und α-Acyloxy-3-indolylessigsäureverbindungen, die an dem Stickstoffatom des indolrings einen von einer aromatischen Carbonsäure abgeleiteten Acylrest, (Aroyl- oder Heteroaroylrest) mit weniger als drei kondensierten Ringen ent-halten, sowie deren anti-inflammatorische Wirkung.

Ziel der vorliegenden Erfindung ist es, neue Verbindungen aus der Indolyl-3-glyoxylsäure-Reihe zur Verfügung zu stellen, die antiasthmatische und immunmoduüerende Wirkung besitzen.

Ferner werden die chemischen Verfahren zur Herstellung dieser Verbindungen sowie galvanische Verfahren zur Überführung der neuen Verbindungen in Arzneimittel und deren Zubereitungsformen beschrieben.

Der Gegenstand der Erfindung umfaßt Verbindungen der allgemeinen Formel I,

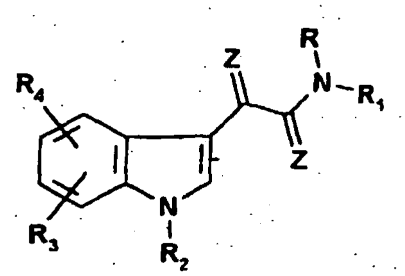

Formel I

wobei die Reste R,$R_1$,$R_2$,$R_3$,$R_4$ und Z folgende Bedeutung haben:

R= Wasserstoff, ($C_1$-$C_6$)-Alkyl, wobei die Alkylgruppe ein- oder mehrfach durch den Phenylring substituiert sein kann. Dieser Phenylring kann seinerseits ein- oder mehrfach durch Halogen, ($C_1$-$C_6$)-Alkyl, ($C_3$-$C_7$)-Cycloalkyl, durch Carboxylgruppen, mit $C_1$-$C_6$-Alkanolen veresterte Carboxylgruppen, Trifluormethylgruppen, Hydroxylgruppen, Methoxygruppen, Ethoxygruppen, Benzyloxygruppen sowie durch eine im Phenylteil ein- oder mehrfach mit ($C_1$-$C_6$)- Alkylgruppen, Halogenatomen oder Trifluormethylgruppen substituierte Benzylgruppe substituiert sein.

$R_1$ kann den Phenylring, der ein- oder mehrfach mit ($C_1$-$C_6$)-Alkyl, ($C_1$-$C_6$)-Alkoxy, Hydroxy, Benzyloxy, Nitro, Amino, ($C_1$-$C_6$)-Alkylamino,($C_1$-$C_6$)-Alkoxycarbonylamino und mit der Carboxylgruppe bzw. der mit $C_1$-$C_6$-Alkanolen veresterten Carboxylgruppe substituiert ist, oder ein Pyridin-Gerüst der Formel II

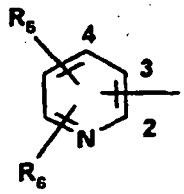

Formel II

bedeuten, wobei das Pyridin-Gerüst wahlweise an den Ringkohlenstoff-Atomen 2,3 und 4 gebunden ist und mit den Substituenten $R_5$ und $R_6$ substituiert sein kann. Die Reste $R_5$ und $R_6$ können gleich oder verschieden sein und die Bedeutung ($C_1$-$C_6$)-Alkyl, sowie die Bedeutung ($C_3$-$C_7$)-Cycloalkyl, ($C_1$-$C_6$)-Alkoxy, Nitro, Amino, Hydroxy, Halogen und Trifluormethyl besitzen und ferner den Ethoxycarbonylamino-Rest sowie die Gruppe Carboxyalkyloxy darstellen, bei dem die Alkylgruppe über 1-4 C-Atome verfügen kann.

$R_1$ kann ferner ein 2-bzw. 4-Pyrimidinyl-Heterocyclus oder ein Pyridylmethyl-Rest, worin $CH_2$ in der 2-, 3-, 4-Stellung stehen kann, sein, wobei der 2-Pyrimidinyl-Ring ein-oder mehrfach mit der Methylgruppe substituiert sein kann, weiterhin das mit ($C_1$-$C_6$)-Alkyl, Halogen, der Nitrogruppe, der Aminogruppe und dem ($C_1$-$C_6$)-Alkyl-amino-Rest substituierte 2-,3- und 4- Chinolylgerüst bedeuten, eine 2-,3- und 4-Chinolylmethylgruppe darstellen, wobei die Ringkohlenstoffe des Pyridylmethyl-und Chinolylmethyl-Restes mit ($C_1$-$C_6$)-Alkyl, ($C_1$-$C_6$)-Alkoxy, Nitro, Amino und ($C_1$-$C_6$)-Alkoxycarbonylamino substituiert sein können.

$R_1$ kann weiterhin für den Fall, daß R Wasserstoff oder die Benzylgruppe bedeuten, der Säurerest einer natürlichen oder unnatürlichen Aminosäure sein, z.B. den α-Glycyl-, den α-Sarkosyl-, den α-Atanyl-, den α-Leucyl-, den α-iso-Leucyl-, den α-Seryl-, den α-Phenylalanyl-, den α-Histidyl-, den α-Prolyl-, den α-Arginyl-, den α-Lysyl-, den α- Asparagyl- und den α-Glutamyl-Rest darstellen, wobei die Amino-gruppen der jeweiligen Aminosäuren ungeschützt vorliegen oder geschützt sein können. Als Schutzgruppe der Aminofunktion kommen der Carbobenzoxy-Rest (Z-Rest) und der tert.-Butoxycarbonyl-Rest (BOC-Rest) sowie die Acetylgruppe in Frage. Im Fall des für $R_1$ beanspruchten Asparagyl- und Glutamylrestes liegt die zweite, nicht gebundene Carboxylgruppe als freie Carboxylgruppe oder in Form eines Esters mit $C_1$-$C_6$-Alkanolen, z.B. als Methyl-, Ethyl- bzw. als tert.- Butylester vor. Weiterhin kann $R_1$ die Allylaminocarbonyl-2-methyl-prop-1-yl-Gruppe bedeuten. R und $R_1$ können ferner zusammen mit dem Stickstoff-Atom, an das sie gebunden sind, einen Piperazinring der Formel III oder einen Homopiperazinring bilden, sofern $R_1$ eine Aminoalkylengruppe darstellt, bei dem

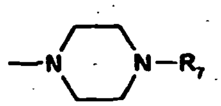

Formel III

$R_7$ einen Alkylrest darstellt, einen Phenylring bedeutet, der ein- oder mehrfach mit ($C_1$-$C_6$)-Alkyl, ($C_1$-$C_6$)-Alkoxy, Halogen, der Nitrogruppe, der Aminofunktion, mit ($C_1$-$C_6$)-Alkylamino, der Benzhydrylgruppe und der Bis-p-fluorbenzylhydrylgruppe substituiert sein kann.

$R_2$ die ($C_1$-$C_6$)-Alkyl-Gruppe ist, wobei die Alkylgruppe ein- oder mehrfach durch Halogen und Phenyl substituiert ist, das seinerseits ein-oder mehrfach durch Halogen, ($C_1$-$C_6$)-Alkyl, ($C_3$-$C_7$)-Cycloalkyl, Carboxylgruppen, mit $C_1$-$C_6$-Alkanolen veresterte Carboxylgruppen, Trifluormethylgruppen, Hydroxylgruppen, Methoxygruppen, Ethoxygruppen oder Benzyloxygruppen substituiert sein kann. Die für $R_2$ geltende ($C_1$-$C_6$)-Alkyl-Gruppe kann ferner durch die 2-Chinolylgruppe und das 2-,3- und 4-Pyridyl-Gerüst substituiert sein, die beide jeweils ein- oder mehrfach durch Halogen, ($C_1$-$C_4$)-Alkylgruppen oder ($C_1$-$C_4$)-Alkoxy-gruppen substituiert sein können.

$R_3$ und $R_4$ können gleich oder verschieden sein und Wasserstoff, Hydroxy, ($C_1$-$C_6$)-Alkyl, ($C_3$-$C_7$)-Cycloalkyl, ($C_1$-$C_6$)-Alkanoyl, ($C_1$-$C_6$)-Alkoxy, Halogen und Benzyloxy bedeuten. Weiterhin können $R_3$ und $R_4$ die

Nitrogruppe, die Aminogruppe, die ($C_1$-$C_4$)-mono- oder dialkylsubstituierte Aminogruppe, und die ($C_1$-$C_3$)-Alkoxy-carbonylamino-Funktion oder ($C_1$-$C_3$)-Alkoxycarbonylamino-($C_1$-$C_3$)-alkyl-Funktion bedeuten.

Z steht für O und S

Unter der Bezeichnung Alkyl-, Alkanol-, Alkoxy- oder Alkylaminogruppe sind für die Reste R, $R_1$, $R_2$, $R_3$, $R_4$, $R_5$, $R_6$, $R_7$ regelmäßig sowohl "geradkettige" als auch "verzweigte" Alkylgruppen zu verstehen, wobei "geradkettige Alkylgruppen" beispielsweise Reste wie Methyl, Ethyl, n-Propyl, n-Butyl, n-Pentyl, n-Hexyl bedeuten können und "verzweigte Alkylgruppen" beispielsweise Reste wie Isopropyl oder tert.-Butyl bezeichnen. Unter "Cycloalkyl" sind Reste wie beispielsweise Cyclopropyl, Cyclobutyl, Cyclopentyl, Cyclohexyl oder Cycloheptyl zu verstehen.
Die Bezeichnung "Halogen" steht für Fluor, Chlor, Brom oder Jod. Die Bezeichnung "Alkoxygruppe" stellt Reste wie beispielsweise Methoxy, Ethoxy, Propoxy, Butoxy, Isopropoxy, Isobutoxy oder Pentoxy dar.

Die erfindungsgemäßen Verbindungen können auch als Säureadditionssalze vorliegen, beispielsweise als Salze von Mineralsäuren, wie beispielsweise Salzsäure, Schwefelsäure, Phosphorsäure, Salze von organischen Säuren, wie beispielsweise Essigsäure, Milchsäure, Malonsäure, Maleinsäure, Fumarsäure, Gluconsäure, Glucuronsäure, Zitronensäure, Embonsäure, Methansulfonsäure, Trifluoressigsäure und Bernsteinsäure.

Sowohl die Verbindungen der Formel I als auch deren Salze sind biologisch aktiv. Die Verbindungen der Formel I können in freier Form oder als Salze mit einer physiologisch verträglichen Säure verabreicht werden.
Die Applikation kann peroral, parenteral, intravenös, transdermal oder inhalativ vorgenommen werden.

Weiterhin betrifft die Erfindung pharmazeutische Zubereitungen mit einem Gehalt an mindestens einer Verbindung der Formel I oder deren Salz mit physiologisch verträglichen anorganischen oder organischen Säuren und gegebenenfalls pharmazeutisch verwendbaren Träger- und/oder Verdünnungs- beziehungsweise Hilfsstoffen.

Als Applikationsformen eignen sich beispielsweise Tabletten, Dragees, Kapseln, Lösungen beziehungsweise Ampullen, Suppositorien, Pflaster, inhalativ einsetzbare Pulverzubereiungen, Suspensionen, Cremes und Salben.

Die erfindungsgemäßen Verbindungen zeigen eine gute antiasthmatische, antiallergische und immunsuppressive/immunmodulierende Wirkung, beispielsweise bei Transplantationen und Krankheiten wie Psoriasis, rheumatoiden Erkrankungen und chronischer Polyarthritis, in den folgenden pharmakologischen Modellen:

**Hemmung der "late phase" - Eosinophilie in der BAL 24 Stunden nach Allergen-Challenge an Meerschweinchen**

Männliche Meerschweinchen (200 - 250 g, Dunkin Hartley Shoe) wurden subcutan mit Ovalbumin (10 µg Ovalbumin + 1 mg $Al(OH)_3$) aktiv sensibilisiert und 2 Wochen später geboostert. Eine Woche nach dem Boostern mit Ovalbumin wurden die Tiere einer inhalativen Challenge mit Ovalbumin (0,5 %ige Lösung) für 20 - 30 Sekunden ausgesetzt. 24 Stunden später wurden die Tiere mittels einer Überdosis Urethan getötet, entblutet un mit 2 x 5 ml 0,9 %iger physiologischer Kochsalzlösung eine bronchoalveoläre Lavage (BAL) durchgeführt.

Die Lavageflüssigkeit wurde gesammelt und 10 Minuten bei 400 g zentrifugiert, das Pellet in 1 ml 0,9 %iger physiologischer Kochsalzlösung suspendiert. Die Eosinophilen wurden nach Färbung mittels Becton Dickinson Testkit Nr. 5877 mikroskopisch in einer Neubauerkammer gezählt. Dieser Testkit enthält Phloxin B als selektiven Farbstoff für Eosinophile. Dabei wurde für jedes Tier die Eosinophilen in der BAL gezählt und als Eosinophile (Millionen/Tier) ausgedrückt. Für jede Gruppe wurden Mittelwert und Standardabweichung bestimmt. Die prozentuale Hemmung der Eosinophilie für die mit Testsubstanz behandelte Gruppe wurde nach folgender Formel berechnet:

$$(A - B) - (B - C) / (A - C) \times 100 = \%\ \text{Hemmung}$$

dabei entsprechen A-Eosinophilen der unbehandelten Challenge-Gruppe, B-Eosinophilen der behandelten Gruppe und C-Eosinophilen der ungechallengten Kontrollgruppe.

Die Tiere wurden zur Vermeidung von Exitus 2 Stunden vor Allergen-Challenge mit einem Histamin $H_1$-Antagonisten (Azelastin; 0,01 mg/kg p.o.) behandelt. Die Applikation der Testsubstanzen oder des Vehikels erfolgte 4 Stunden nach Allergen-Challenge. Die prozentuale Hemmung der Eosinophilie in der BAL wurde an Gruppen von 6 - 10 Tieren errechnet.

Tabelle: Hemmung der "late phase" - Eosinophilie 24 h nach Allergen-Challenge an Meerschweinchen

| Substanz | Dosis [mg/kg] | Applikation | n | % Hemmung |
|---|---|---|---|---|
| **Cyclosporin A** | 5 | i.p. + 4h | 17 | 50,0 |
| | 10 | i.p. + 4h | 11 | 47,0 |
| | 30 | p.o. + 4h | 10 | 68,8 |
| **gemäß Bsp. 1** | 5 | i.p. + 4h | 10 | 27,8 |
| | 10 | i.p. + 4h | 10 | 55,4 |
| | 30 | p.o. + 4h | 9 | 56,1 |

**Assays zur Bestimmung der Peptidylprolylisomerase (PPlase)-Aktivität und Hemmung**

Die PPlase-Aktivität der Cyclophiline wurde enzymatisch nach Fischer et. al. (1984) gemessen. Nach Isomerisierung des Substrates durch die Peptidylprolylisomerase, ist dieses für Chymotrypsin zugänglich, das das Chromophor p-Nitroaniline spaltet. Für die Bestimmung der Hemmung der PPiase-Aktivität durch Substanz wurde rekombinantes humanes Cyp B verwendet. Die Interaktion von Cyp B mit einem potentiellen Inhibitor wurde wie folgt durchgeführt: Eine bestimmte Konzentration gereinigten Cyp B wurde mit 1 μM Substanz für 15 min inkubiert. Die PPlase-Reaktion wurde durch Zugabe der Substratlösung zum Reaktionsgemisch gestartet, das HEPES-Puffer, Chymotrypsin und entweder Test- oder Kontrollproben enthält. Unter diesen Bedingungen wurde eine Kinetik erster Ordnung erhalten mit einer Konstanten $K_{beobachtet} = K_0 + K_{enz}$, wobei $K_0$ die Spontanisomerisierung und $K_{enz}$ die Geschwindigkeit der Isomerisierung der PPlase-Aktivität ist. Die Extinktionswerte, die der Menge des gespaltenen Chromophors entsprechen, wurden mit einem Beckman DU 70 Spektrophotometer bei einer konstanten Reaktionstemperatur von 10 ˚C gemessen. Die beobachtete Restaktivität in Gegenwart verschiedener Substanzen wurde mit den nur mit Lösungsmittel behandelten Cyclophilinen verglichen. Die Ergebnisse wurden in % Restaktivität angegeben. Cyclosporin A (CsA) wurde als Referenzverbindung verwendet. Zusätzlich wurde die Hemmung der PPlase-Aktivität durch SDS-PAGE kontrolliert.

**Kolorimetrisches Assay (auf dem MTT-Test basierend) für die nicht-radioaktive Quantifizierung der Zellproli-** feration und **Überlebensfähigkeit**

MTT wird für die quantitative Bestimmung der Zellproliferation und Aktivierung z. B. bei der Reaktion auf Wachstumsfaktoren und Cytokine wie IL-2 und IL-4 verwendet sowie für die Quantifizierung der antiproliferativen oder toxischen Wirkungen.

Der Assay basiert auf der Spaltung von gelbem Tetrazoliumsalz MTT zu purpurroten Formazankristallen durch metabolisch aktive Zellen.

Die Zellen, in einer 96 Loch-Gewebekulturplatte gezüchtet, werden mit gelber MTT-Lösung für ca. 4h inkubiert. Nach dieser Inkubationszeit bilden sich purpurrote Formazansalzkristalle. Diese Salzkristalle sind in wässrigen Lösungen unlöslich, können aber durch Zugabe von Lösungsvermittler und durch Inkubation der Platten über Nacht gelöst werden.

Das gelöste Formazanprodukt wird spektrophotometrisch unter Verwendung eines ELISA-Readers quantifiziert Ein Anstieg der Zahl lebender Zellen resultiert in einem Anstieg der gesamtmetabolischen Aktivität in der Probe. Dieser Anstieg korreliert direkt mit der Menge der gebildeten purpurroten Formazankristalle, die durch die Absorption gemessen werden.

| **Substanz** | **Hemmung der PPlase-Aktivität** [%] | **Hemmung der CD3- induz. IL-2-Produktion** [%] | | | **Hemmung der Lyinphoproliferation** [%] | | |
|---|---|---|---|---|---|---|---|
| Konz. [μM] | | 0,1 | 1 | 10 | 0,1 | 1 | 10 |
| gemäß Bsp. 1 | 80 - 100 | 34 | 72 | 95 | 18 | 39 | 61 |
| Cyclosporin A | 80 - 100 | 56 | 82 | 94 | 8 | 7 | 11 |

Die Verfahren zur Herstellung der erfindungsgemäßen Verbindungen werden in den folgenden Reaktionsschemata 1 und 2 sowie in allgemeinen Vorschriften beschrieben. Alle Verbindungen lassen sich wie beschrieben oder analog herstellen.

Die Verbindungen der allgemeinen Formel 1 sind gemäß des folgenden Schemas 1 erhältlich, dargestellt am Aufbau der Verbindung Beispiel 1:

<u>Schema 1</u>

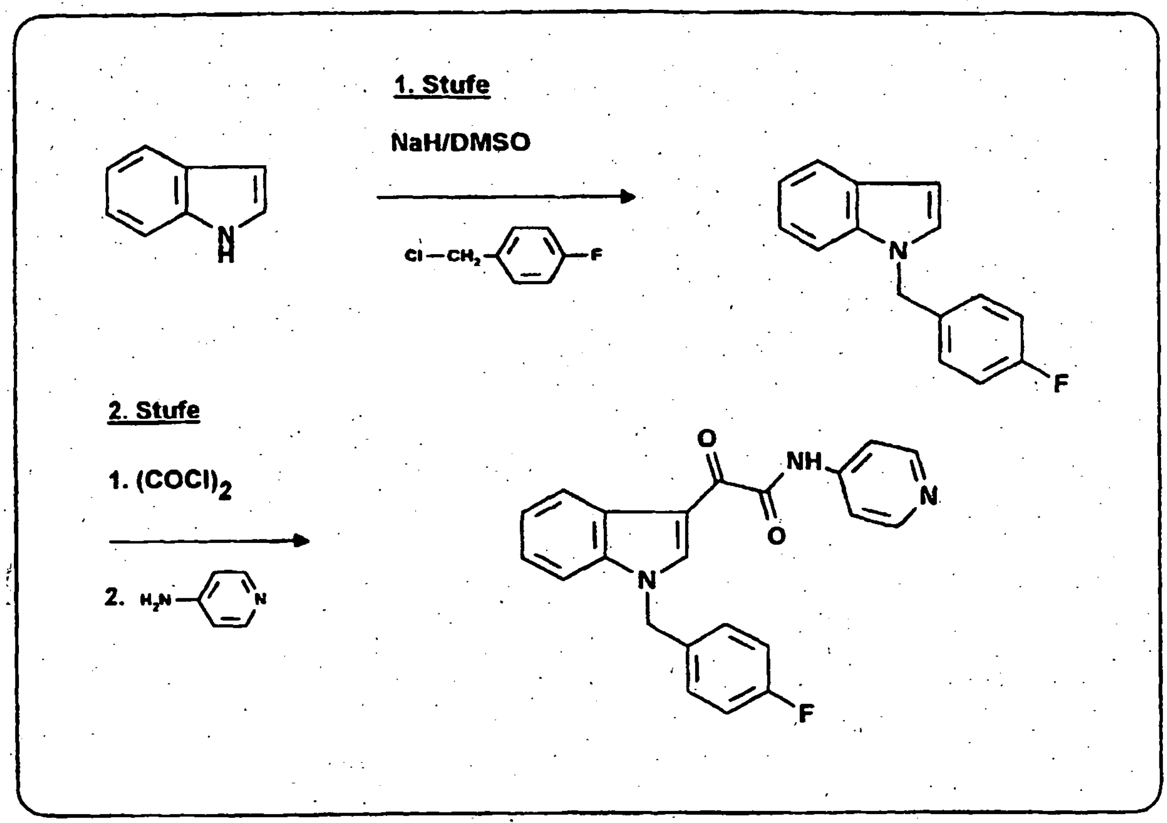


<u>Allgemeine Vorschrift zur Darstellung der Verbindungen der allgemeinen Formel I gemäß Schema 1:</u>

<u>1. Stufe:</u>

Das Indol-Derivat, das unsubstituiertoderan C-2 oder im Phenylgerüst einfach oder mehrfach substituiert sein kann, wird in einem protischen, dipolar aprotischen oder unpolaren organischen Lösungsmittel, wie beispielsweise Isopropanol, Tetrahydrofuran, Dimethylsulfoxid, Dimethylfortnamid, Dimethylacetamid, N-Methylpyrrolidon, Dioxan, Toluol oder Methylenchlorid gelöst und tropfenweise zu einer in einer Dreihalskolben unter $N_2$-Atmosphäre vorbereiteten molaren oder überschüssig eingesetzten Suspension einer Base, wie beispielsweise Natriumhydrid, pulversiertes Kaliumhydroxid, Kalium-tert.-butylat, Dimethylaminopyridin oder Natriumamid in einem geeigneten Lösungsmittel gegeben. Sodann gibt man beispielsweise das gewünschte Alkyl-, Aralkyl-bzw. Heteroaralkylhalogenid gegebenenfalls unter Zusatz eines Katalysators, wie z.B. Kupfer, zu und läßt einige Zeit, beispielsweise 30 Minuten bis 12 Stunden, nachreagieren und hält die Temperatur innerhalb eines Bereichs von 0°C bis 120°C, vorzugsweise zwischen 30°C bis 80°C, besonders zwischen 50°C und 65°C. Nach Beendigung der Reaktion wird die Reaktionsmischung in Wasser gegeben, die Lösung z.B. mit Diethylether, Dichlormethan, Chloroform, Methyl-tert.-butylether oder Tetrahydrofuran extrahiert und die jeweils erhaltene organische Phase mit wasserfreiem Natriumsulfat getrocknet. Man engt die organische Phase im Vakuum ein, kristallisiert den verbleibenden Rückstand durch Anreiben oder reinigt den öligen Rückstand durch Umkristallisation, Destillation oder durch Säulen- bzw. Flash-Chromatographie an Kieselgel oder Aluminiumoxid. Als Laufmittel dient beispielsweise ein Gemisch aus Dichlormethan und Diethylether im Verhältnis 8:2 (Vol/Vol) oder ein

Gemisch aus Dichlormethan und Ethanol im Verhältnis 9:1 (Vol/Vol).

2. Stufe

Das nach obenstehender Vorschrift der 1. Stufe erhaltene N-substituierte Indol wird unter Stickstoffatmosphäre in einem aprotischen oder unpolaren organischen Lösungsmittel, wie beispielsweise Diethylether, Methyl-tert.-butylether, Tetrahydrofuran, Dioxan, Toluol, Xylol, Methylenchlorid oder Chloroform gelöst und zu einer unter Stickstoff-Atmosphäre bereiteten Lösung einer einfach molaren bis zu 60-prozentig überschüssigen Menge Oxalylchlorid in einem aprotischen oder unpolaren Lösungsmittel, wie z.B. in Diethylether, Methyl-tert.-butylether, Tetrahydrofuran, Dioxan, Toluol, Xylol, Methylenchlorid oder Chloroform gegeben, wobei die Temperatur zwischen -5˚C und 20˚C gehalten wird. Man erhitzt sodann die Reaktionslösung bei einer Temperatur zwischen 10˚C und 130˚C, vorzugsweise zwischen 20˚C und 80˚C, besonders zwischen 30˚C und 50˚C für einen Zeitraum von 30 Minuten bis zu 5 Stunden und dampft anschließend das Lösungsmittel ab. Der verbleibende Rückstand des auf diese Weise gebildeten "Indolyl-3-glyoxylsäurechlorids" wird in einem aprotischen Lösungsmittel wie z.B. Tetrahydrofuran, Dioxan, Diethylether, Toluol oder auch in einem dipolar aprotischen Lösungsmittel, wie z.B. Dimethylformamid, Dimethylacetamid oder Dimethylsulfoxid gelöst, auf eine Temperatur zwischen 10˚C und -15˚C, vorzugsweise zwischen -5˚C und 0˚C, gekühlt und in Gegenwart eines Säurefängers mit einer Lösung des primären oder sekundären Amins in einem Verdünnungsmittel versetzt.

Als Verdünnungsmittel kommen die oben zur Auflösung des Indolyl-3-glyoxylsäurechlorids verwendeten Lösungsmittel in Frage. Als Säurefänger finden Triethylamin, Pyridin, Dimethylaminopyridin, bas. Ionenaustauscher, Natriumcarbonat, Kaliumcarbonat, pulverisiertes Kaliumhydroxid sowie überschüssiges, zur Reaktion eingesetztes, primäres oder sekundäres Amin Verwendung. Die Reaktion findet bei einer Temperatur von 0˚C bis 120˚C, vorzugsweise bei 20-80˚C besonders zwischen 40˚C und 60˚C statt. Nach 1-3 stündiger Reaktionszeit und 24-stündigem Stehen bei Raumtemperatur wird das Hydrochlorid des Säurefängers filtriert, das Filtrat i.Vak. eingeengt und der Rückstand aus einem organischen Lösungsmittel umkristallisiert oder durch Säulenchromatographie über Kieselgel oderAluminiumoxid gereinigt. Als Laufmittel findet z.B. ein Gemisch aus Dichlormethan und Ethanol (95:5, Vol/Vol) Verwendung.

**Ausführungsbeispiele**

Gemäß dieser allgemeinen Vorschrift für die Stufen 1 und 2 , denen das Syntheseschema 1 zugrundeliegt, wurden folgende Verbindungen synthetisiert, die unter Angabe der jeweiligen chemischen Bezeichnung aus der nachfolgenden Übersicht hervorgehen. In der sich anschließenden Tabelle 1 sind aus der allgemeinen Formel 1 und den Substituenten $R_1$-$R_4$ und Z die Stukturen dieser Verbindungen und ihre Schmelzpunkte zu ersehen:

**Beispiel 1**

N-(Pyridin-4-yl)-[1-(4-fluorbenzyl)-indol-3-yl]glyoxylamid

1. Stufe

1-(4-Fluorbenzyl)-indol

In eine Mischung von 2,64 g Natriumhydrid (0,11 Mol, Mineralölsuspension) in 100 ml Dimethylsulfoxid wird eine Lösung von 11,72 g (0,1 Mol) Indol in 50 ml Dimethylsulfoxid gegeben. Man erhitzt 1,5 Stunden auf 60˚C, läßt danach abkühlen und tropft 15,9 g (0,11 Mol) 4-Fluorbenzylchlorid zu. Die Lösung wird auf 60˚C erwärmt; über Nacht stehengelassen und sodann unter Rühren in 400 ml Wasser gegossen. Man extrahiert mehrmals mit insgesamt 150 ml Methylenchlorid, trocknet die organische Phase mit wasserfreiem Natriumsulfat, filtriert und engt das Fittrat i.Vak. ein. Der Rückstand wird i. Hochvakuum destilliert: 21,0 g (96% d.Th.)
Sdp. (0,5mm): 140˚C

2. Stufe

N-(Pyridin-4-yl)-[1-(4-fluorbenzyl)-indol-3-yl] glyoxylamid

Zu einer Lösung von 2,25 ml Oxalylchlorid in 25 ml Ether wird bei 0˚C und unter $N_2$ tropfenweise eine Lösung von 4,75 g (21,1 mMol) 1-(4-Fluorbenzyl)-indol in 25 ml Ether gegeben. Man erhitzt 2 Stunden zum Rückfluß und dampft anschließend das Lösungsmittel ab. Sodann wurden zum Rückstand 50 ml Tetrahydrofuran zugefügt, die Lösung auf -5˚C abgekühlt und tropfenweise mit einer Lösung von 4,66 g (49,5 mMol) 4-Aminopyridin in 200 ml THF versetzt. Man erhitzt 3 Stunden zum Rückfluß und läßt über Nacht bei Raumtemperatur stehen. Das 4-Aminopyridin Hydrochlorid wird

abgesaugt, der Niederschlag mit THF gewaschen, das Filtrat i. Vak.eingeengt und der Rückstand aus Essigester umkristallisiert.
Ausbeute: 7,09 g (90% d.Th.)
Schmelzpunkt: 225-2262˚C

| Elementaranalyse: | | | | | | |
|---|---|---|---|---|---|---|
| ber. | C | 70,77 | H | 4,32 | N | 11,25 |
| gef. | C | 71,09 | H | 4,36 | N | 11,26 |

| | |
|---|---|
| Beispiel 2 | N-(Pyridin-4-yl)-(1-methyl-indol-3-yl) glyoxylamid |
| Beispiel 3 | N-(Pyridin-3-yl)-[1-(4-fluorbenzyl)-indol-3-yl]-glyoxylamid |
| Beispiel 4. | N-(Pyridin-3-yl)-(1-benzylindol-3-yl)-glyoxylamid |
| Beispiel 5 | N-(Pyridin-3-yl)-[1-(2-chlorbenzyl)-indol-3-yl]-glyoxylamid |
| Beispiel 6 | N-(4-Fluorphenyl)-[1-(4-fluorbenzyl)-indol-3-yl]-glyoxylamid |
| Beispiel 7 | N-(4-Nitrophenyl)-[1-(4-fluorbenzyl)-indol-3-yl]-glyoxylamid |
| Beispiel 8 | N-(2-Chlorpyridin-3-yl)-[1-(4-fluorbenzyl)-indol-3-yl]-glyoxylamid |
| Beispiel 9 | N-(Pyridin-4-yl)-(1-benzylindol-3-yl)-glyoxylamid |
| Beispiel 10 | N-(Pyidin-4-yl)-[1-(3-pyridylmethyl)-indol-3-yl]-glyoxylamid |
| Beispiel 11 | N-(4-Fluorphenyl)-[1-(2-pyridylmethyl)-indol-3-yl]-glyoxylamid |
| Beispiel 12 | N-(4-Fluorphenyl)-[1-(3-pyridylmethyl)-indol-3-yl]-glyoxylamid |
| Beispiel 13 | N-(Pyridin-4-yl)-[1-(4-chlorbenzyl)-indol-3-yl]-glyoxylamid |
| Beispiel 14 | N-(Pyridin-4-yl)-[1-(2-chlorbenzyl)-indol-3-yl]-glyoxylamid |
| Beispiel 15 | N-(Pyridin-2-yl)-[1-(4-fluorbenzyl)-indol-3-yl]-glyoxylamid |
| Beispiel 16 | N-(Pyridin-4-yl)-[1-(2-pyridylmethyl)-indol-3-yl]-glyoxylamid |
| Beispiel 17 | (4-Phenyl-pipenazin-1-yl)-[1-(4-fluorbenzyl)-indol-3-yl]-glyoxylamid |
| Beispiel 18 | N-(Pyridin-2-yl)-(1-benzyl-indol-3-yl)-glyoxylamid |
| Beispiel 19 | N-(Pyridin-4-yl)-[1-(4-fluorbenzyl)-6-ethoxycarbonylamino-indol-3-yl]-glyoxylamid |
| Beispiel 20 | N-(Pyridin-4-yl)-[1-(4-fluorbenzyl)-5-ethoxycarbonylamino-indol-3-yl]-glyoxylamid |
| Beispiel 21 | N-(Pyridin-4-yl)-[1-(4-fluorbenzyl)-6-cyclopentyloxycarbonylamino-indol-3-yl]-glyoxylamid |
| Beispiel 22 | 4-(Pyridin-4-yl)-piperazin-1-yl)-[1-(4-fluorbenzyl)-indol-3-yl]-glyoxylamid |
| Beispiel 23 | N-(3,4,5-Trimethoxybenzyl)-N-(allylaminocarbonyl-2-methyl-prop-1-yl)-[1-(4-fluorbenzyl)-indol-3-yl]-glyoxylamid |
| Beispiel 24 | N-(Pyridin-4-yl)-[1-(4-fluorbenzyl)-5-methoxy-indol-3-yl]-glyoxylamid |
| Beispiel 25 | N-(Pyridin-4-yl)-[1-(4-fluorbenzyl)-5-hydroxy-indol-3-yl]-glyoxylamid |
| Beispiel 26 | N-(Pyridin-4-yl-[1-(4-fluorbenzyl)-5-ethoxycarbonylamino-methyl-indol-3-yl]-glyoxylamid |

**Tabelle 1 : Neue Indolylglyoxylamide gemäß Reaktionsschema 1**

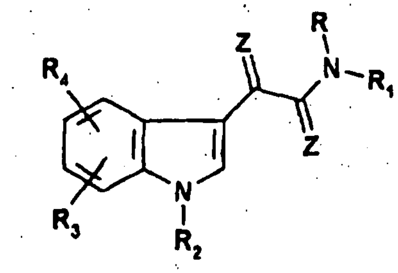

Formel I

| Beispiel | R | $R_1$ | $R_2$ | $R_3$ | $R_4$ | Z | Fp. |
|---|---|---|---|---|---|---|---|
| Bsp.1 | **H** | 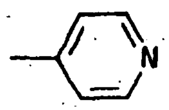 | 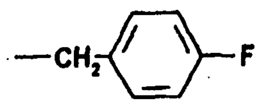 | H | H | O | 225-6°C |
| Bsp.2 | H | 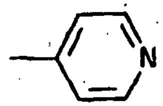 | $CH_3$ | H | H | O | 176°C |
| Bsp.3 | H | 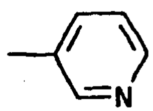 | 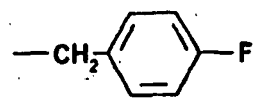 | H | H | O | 173°C |
| Bsp.4 | H | 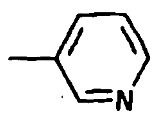 | 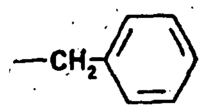 | H | H | O | 140°C |
| Bsp.5 | H | 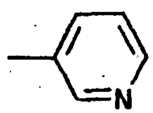 | 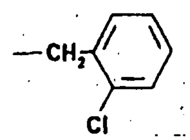 | H | H | O | 185°C |

(fortgesetzt)

| Beispiel | R | $R_1$ | $R_2$ | $R_3$ | $R_4$ | Z | Fp. |
|---|---|---|---|---|---|---|---|
| Bsp.6 | H | 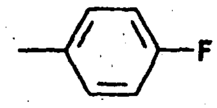 | 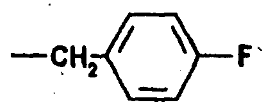 | H | H | O | 199°C |
| Bsp.7 | H | 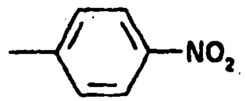 | 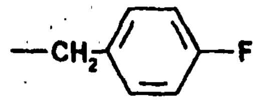 | H | H | O | >250°C |
| Bsp.8 | H | 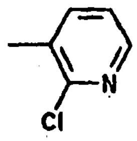 | 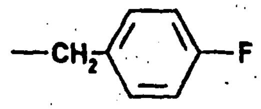 | H | H | O | 149°C |
| Bsp.9 | H | 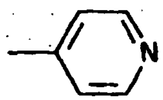 | 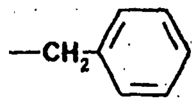 | H | H | O | 178-180°C |
| Bsp. 10 | H | 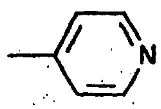 | 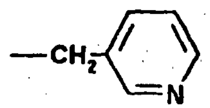 | H | H | O | 179°C |
| Bsp. 11 | H | 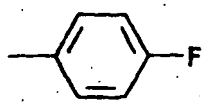 | 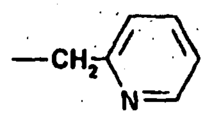 | H | H | O | 132°C |
| Bsp. 12 | H | 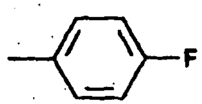 | 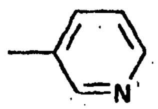 | H | H | O | 144°C |
| Bsp. 13 | H | 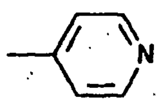 | 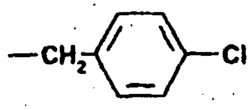 | H | H | O | 234°C |

(fortgesetzt)

| Beispiel | R | $R_1$ | $R_2$ | $R_3$ | $R_4$ | Z | Fp. |
|---|---|---|---|---|---|---|---|
| Bsp.14 | H | 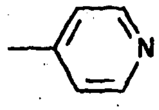 | 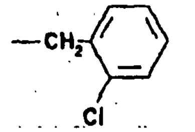 | H | H | O | 184°C |
| Bsp. 15 | H | 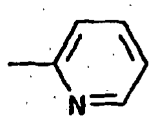 | 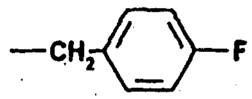 | H | H | O | 141°C |
| Bsp. 16 | H | 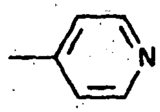 | 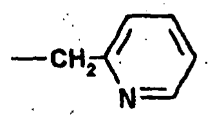 | H | H | O | 202°C |
| Bsp. 17 | $R+R_1$ zusam. | 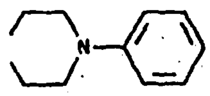 | 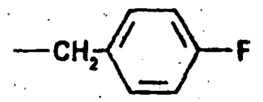 | H | H | O | 115°C |
| Bsp. 18 | H | 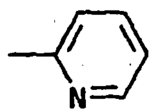 | 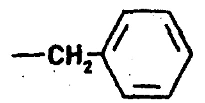 | H | H | O | 112-3°C |
| Bsp. 19 | H | 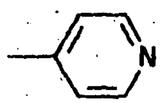 | 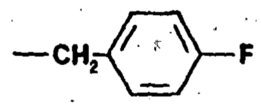 | 6-NHCOOEt | H | O | >250°C |
| Bsp.20 | H | 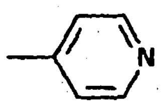 | 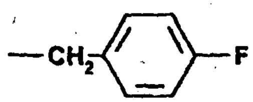 | 5-NHCOOEt | H | O | 183°C |
| Bsp. 21 | H | 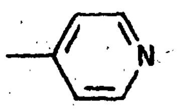 | 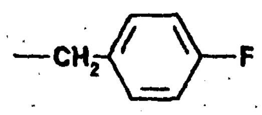 | 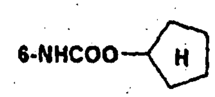 | H | O | Ölig |

(fortgesetzt)

| Beispiel | R | $R_1$ | $R_2$ | $R_3$ | $R_4$ | Z | Fp. |
|---|---|---|---|---|---|---|---|
| Bsp. 22 | R+$R_1$ zusam. | 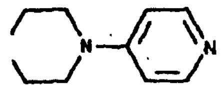 | 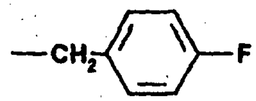 | H | H | O | 160-62°C |
| Bsp. 23 | 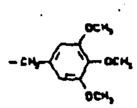 | 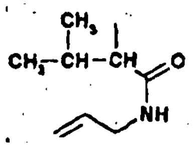 | 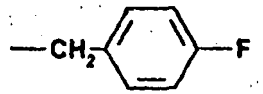 | H | H | O | 139-141°C |
| Bsp. 24 | H | 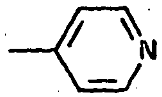 | 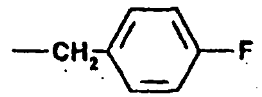 | 5-$OCH_3$ | H | O | 188°C |
| Bsp. 25 | H | 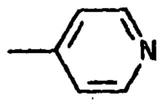 | 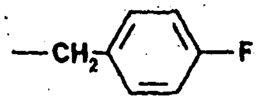 | 5-OH | H | O | >250°C |
| Bsp. 26 | H | 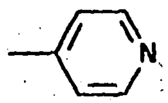 | 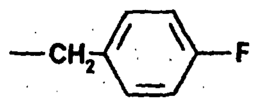 | 5-$CH_2$-NHCOOEt | H | O | 175-178°C |

Ausgangsstufen für die nach Syntheseschema 1 hergestellten Verbindungen der allgemeinen Formel 1, die aus Tabelle 1 hervorgehen.

Für die Syntheseendstufen der Beispiele 1 bis 22 und 24 bis 26 sind alle Vorstufen käuflich.

Weiterhin sind die Verbindungen der allgemeinen Formel 1 auch nach dem Syntheseweg des Schemas 2 erhältlich, dargestellt am Aufbau der Verbindung Beispiel 27:

Schema 2

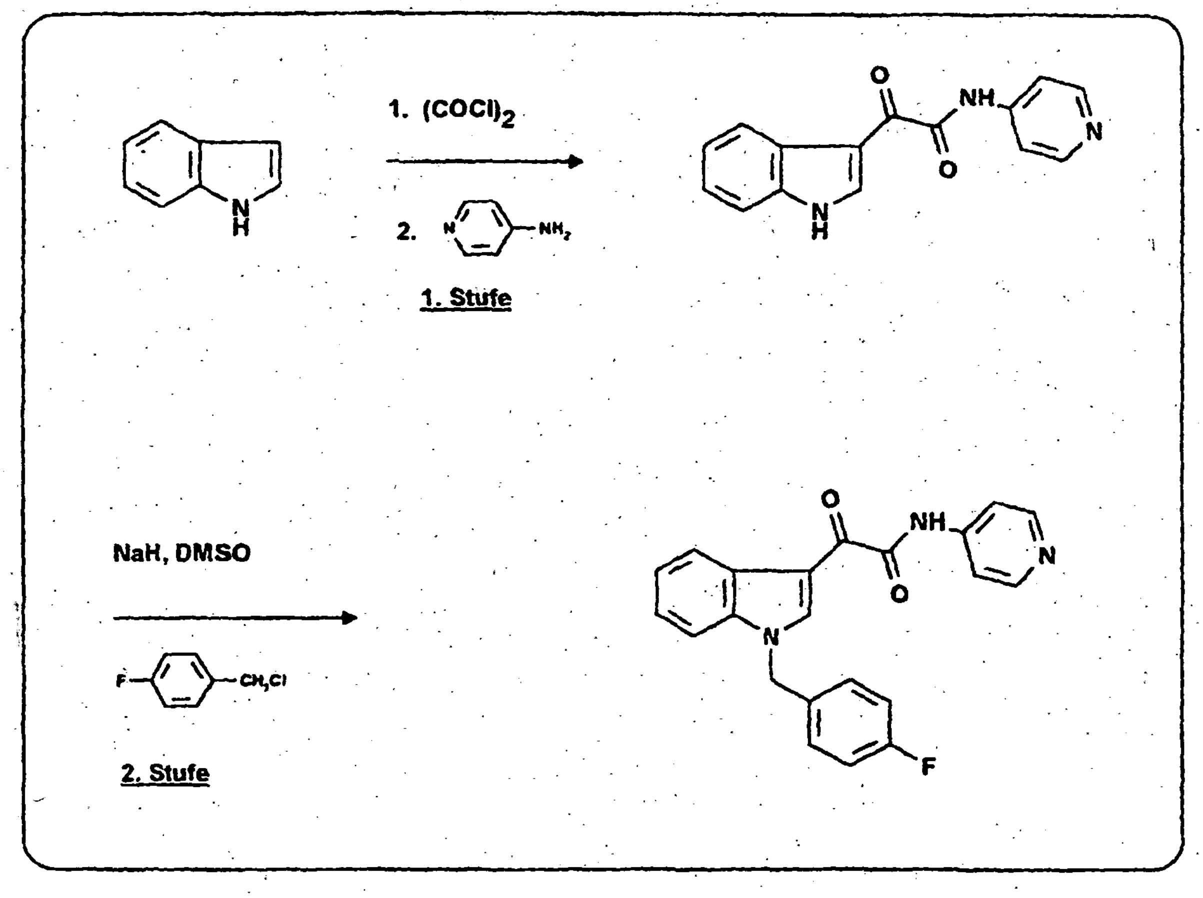


Allgemeine Vorschrift zur Darstellung der Verbindungen der allgemeinen Formel 1 gemäß Schema 2

1. Stufe:

Zu einer unter Stickstoffatmosphäre bereiteten Lösung einer einfach molaren bis zu 60% überschüssigen Menge Oxalylchlorid in einem aprotischen oder unpolaren Lösungsmittel, wie z.B. in Diethylether, Methyl-tert.-butylether, Tetrahydrofuran, Dioxan oder auch Dichlormethan, wird bei einer Temperatur zwischen -5°C und +5°C tropfenweise das in einem Lösungsmittel, wie z.B. oben für Oxalylchlorid angegeben, gelöste Indol-Derivat, das unsubstituiert oder an C-2 bzw. im Phenylring substituiert sein kann, zugegeben. Man erhitzt sodann die Reaktionslösung für 1 bis zu 5 Stunden auf eine Temperatur zwischen 10°C und 120°C, vorzugsweise zwischen 20°C und 80°C, besonders zwischen 30°C und 60°C und dampft anschließend das Lösungsmittel ab. Der verbleibende Rückstand des (Indol-3-yl) glyoxylsäurechlorid wird in einem aprotischen Lösungsmittel, wie z.B. Tetrahydrofuran, Dioxan, Diethylether, Toluol oder auch in einem dipolar aprotischen Lösungsmittel, wie z.B. Dimethylformamid, Dimethylacetamid oder Dimethylsulfoxid gelöst bzw. suspendiert, auf eine Temperatur zwischen -10°C und +10°C, vorzugsweise auf -5°C bis 0°C gekühlt und in Gegenwart eines Säurefängers mit einer Lösung des primären oder sekundären Amins in einem Verdünnungsmittel versetzt. Als Verdünnungsmittel kommen die zur Auflösung des "Indolyl-3-glyoxylsäurechlorids" verwendeten Lösungsmittel in Frage. Als Säurefänger finden Triethylamin, Pyridin, Dimethylaminopyridin bas. Ionenaustauscher, Natriumcarbonat, Kaliumcarbonat, pulverisiertes Kaliumhydroxid sowie überschüssiges, zur Reaktion eingesetztes primäres oder sekundäres Amin Verwendung. Die Reaktion findet bei einer Temperatur von 0˚C bis 120˚C, vorzugsweise bei 20-80C, besonders zwischen 40˚C und 60˚C statt. Nach 1-4-stündiger Reaktionszeit und 24-stündigem Stehen bei Raumtemperaturwird filtriert, der Niederschlag mit Wasser digeriert, abgesaugt und i. Vak. getrocknet. Man reinigt die gewünschte Verbindung durch Umkristallisation in einem organischen Lösungsmittel oderdurch Säulenchromatographie an Kieselgel oder Aluminiumoxid. Als Laufmittel findet z.B. ein Gemisch aus Dichlormethan und Ethanol (10:1, vol/vol) Verwendung.

2. Stufe

Das nach obenstehender Vorschrift der 1. Stufe erhaltene "Indol-3-yl-glyoxylamid" wird in einem protischen, dipolar aprotischen oder unpolaren organischen Lösungsmittel, wie z.B. in Isopropanol, Tetrahydrofuran, Dimethylsulfoxid, Dimethylformamid, Dimethylacetamid, N-Methylpyrrolidon, Dioxan, Toluol oder Methylenchlorid gelöst und tropfenweise zu einer in einem Dreihalskolben unter $N_2$-Atmosphäre vorbereiten molaren oder überschüssig eingesetzten Suspension einer Base, wie z.B. Natriumhydrid, pulverisiertes Kaliumhydroxid, Kalium-tert.-butylat, Dimethylaminopyridin oder Natriumamid in einem geeigneten Lösungsmittel gegeben. Sodann gibt man das gewünschte Alkyl-, Aralkyl- bzw. Heteroaralkylhalogenid entweder unverdünnt oder in einem Verdünnungsmittel, das z.B. auch zur Lösung des "Indol-3-yl-glyoxylamids" verwendet wurde, gegebenenfalls unter Zusatz eines Katalysators, wie z.B. Kupfer, zu und läßt einige Zeit, z.B. 30 Minuten bis 12 Stunden, reagieren und hält die Temperatur innerhalb eines Bereichs zwischen 0˚C und 120˚C, vorzugsweise zwischen 30˚C und 80˚C, besonders zwischen 50 und 70˚C. Nach Beendigung der Reaktion wird das Reaktionsgemisch in Wasser gegeben, die Lösung z.B. mit Diethylether, Dichlormethan, Chloroform, Methyl-tert.-butylether, Tetrahydrofuran bzw. n-Butanol extrahiert und die jeweils erhaltene organische Phase mit wasserfreiem Natriumsulfat getrocknet.
Man engt die organische Phase im Vakuum ein, kristallisiert den verbleibenden Rückstand durch Anreiben bzw. reinigt den öligen Rückstand durch Destillation oder durch Säulen- bzw. Flashchromatographie an Kieselgel oder Aluminiumoxid. Als Laufmittel dient beispielsweise ein Gemisch aus Methylenchlorid und Diethylether im Verhältnis 8:2 (Vol/Vol) oder ein Gemisch aus Methylenchlorid und Ethanol im Verhältnis 9:1 (V/V)

**Ausführungsbeispiele**

Gemäß dieser allgemeinen Vorschrift für die Stufen 1 und 2, denen das Syntheseschema 2 zugrundeliegt, wurden Verbindungen synthetisiert, die auch schon gemäß des Syntheseablaufs des Reaktionsschemas 1 dargestellt wurden und aus Tabelle 1 hervorgehen. Die diesbezüglichen Vorstufen dieser Verbindungen sind aus Tabelle 2 ersichtlich.

**Beispiel 27**

N-(Pyridin-4-yl)-[1-(4-fluorbenzyl)-indol-3-yl]glyoxylamid (Endstufe, mit Beispiel 1 identisch)

1. Stufen

N-(Pyridin-4-yl)-(indol-3-yl)glyoxylamid

Zu einer Lösung von 9 ml Oxalylchlorid in 100 ml wasserfreiem Ether wird tropfenweise bei 0˚C eine Lösung von 10 g (85.3 mMol) Indol in 100 ml Ether zugegeben. Man hält das Gemisch 3 Stunden unter Rückfluß. Sodann wird bei -5˚C eine Suspension von 12 g (127,9 mMol) 4-Aminopyridin in 500 ml Tetrahydrofuran zugetropft, das Reaktionsgemisch unter Rühren 3 Stunden auf Rückflußtemperatur erhitzt und über Nacht bei Raumtemp. stehengelassen. Man filtrierte, behandelte den Niederschlag mit Wasser und reinigte die getrocknete Verbindung über eine Kieselgelsäule (Kieselgel 60, Fa. Merck AG, Darmstadt) unter Anwendung des Elutionsmittels Methylenchlorid/Ethanol (10:1, v/v).
Ausbeute: 9,8g (43,3% d.Th.)
Fp.: ab 250˚C

2. Stufe:

N-(Pyridin-4-yl)-[1-[4-fluorbenzyl)-indol-3-yl]glyoxylamid

Das nach der 1. Stufe erhaltene N-(Pyridin-4-yl)-(indol-3-yl)glyoxylamid wird gemäß der "Benzytierungsvorschrift" (Seite 11) mit 4-Fluorbenzylchlorid umgesetzt und die erhaltene Verbindung isoliert.
Ausbeute: 41% d.Th.
Schmp.: 224-225˚C

| Elementaranalyse: | Ber. C 70,77 | H 4,32 | N 11,25 |
|---|---|---|---|
| | Gef. C 70,98 | H 4,40 | N 11,49 |

Beispiel 28 N-(4-Nitrophenyl)-[1-(4-fluorbenzyl)-indol-3-yl]-glyoxylamid (Endstufe, mit Beispiel 7 identisch)
Beispiel29 N-(4-Fluorphenyl)-[1-(4-fluorbenzyl)-indol-3-yl]-glyoxylamid (Endstufe, mit Beispiel 6 identisch)
Beispiel 30 N-(Pyridin-3-yl)-[1-4-fluorbenzyl)-indol-3-yl]-glyoxylamid (Endstufe, mit Beispiel 3 identisch)

Nach dem vorliegenden Schema 2 wurden die folgenden Vorstufen (1. Stufe des Reaktionsschemas 2, Tabelle 2) erhalten.

Beispiel 31 N-(Pyridin-4-yl)-(indol-3-yl)glyoxylamid
Beispiel 32 N-(4-Nitrophenyl)-(indol-3-yl)glyoxylamid
Beispiel 33 N-(4-Fluorphenyl)-(indol-3-yl)glyoxyamid
Beispiel 34 N-(Pyridin-3-yl)-(indol-3-yl)glyoxylamid

**Tabelle 2: Neue Indolylglyoxylamide gemäß Reaktionsschema 2**

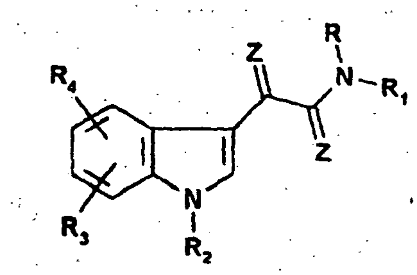

**Formel I**

| **Beispiel** | **R** | **$R_1$** | **$R_2$** | **$R_3$** | **$R_4$** | **Z** | **Fp.** |
|---|---|---|---|---|---|---|---|
| Bsp. 31 | H | 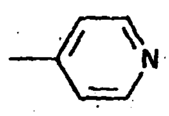 | H | H | H | O | >250°C |
| Bsp.32 | H | 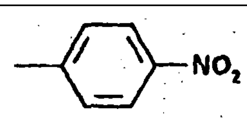 | H | H | H | O | >250°C |
| Bsp. 33 | H | 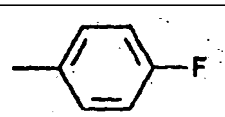 | H | H | H | O | 233-5°C |
| Bsp. 34 | H | 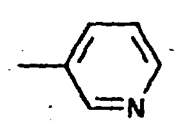 | H | H | H | O | 235°C |

## Patentansprüche

**1.** N-substituierte Indol-3-glyoxylamide der Formel I

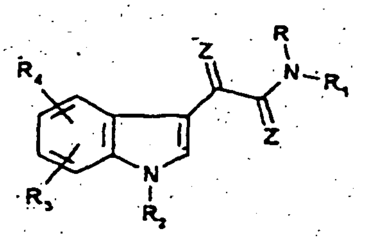

I

sowie deren Säureadditionssalze,
wobei die Reste R, $R_1$, $R_2$, $R_3$, $R_4$ und Z folgende Bedeutung haben:

R= Wasserstoff, ($C_1$-$C_6$)-Alkyl, wobei die Alkylgruppe ein- oder mehrfach durch den Phenylring substituiert sein kann, wobei dieser Phenylring seinerseits ein- oder mehrfach durch Halogen, ($C_1$-$C_6$)-Alkyl, ($C_3$-$C_7$)-Cycloalkyl, durch Carboxylgruppen, mit $C_1$-$C_6$-Alkanolen veresterte Carboxylgruppen. Trifluormethylgruppen, Hydroxylgruppen, Methoxygruppen, Ethoxygruppen. Benzyloxygruppen sowie durch eine im Phenyltell ein- oder mehrfach mit ($C_1$-$C_6$)- Alkylgruppen, Halogenatomen oder Trifluormethylgruppen substituierte Benylgruppe substituiert sein kann,
$R_1$ kann den Phenylring, der ein- oder mehrfach mit ($C_1$-$C_6$)-Alkyl, ($C_1$-$C_6$)Alkoxy, Hydroxy, Benzyloxy, Nitro, Amino, ($C_1$-$C_6$)-Alkylamino,($C_1$-$C_6$)-Alkoxycarbonylamino und mit der Carboxylgruppe bzw. der mit $C_1$-$C_6$-Alkanolen veresterten Carboxylgruppe substituiert ist, oder ein Pyridin-Gerüst der Formel II

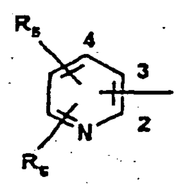

II

bedeuten, wobei das Pyridin-Gerüst wahlweise an den Ringkohlenstoff-Atomen 2,3 und 4 gebunden ist und mit den Substituenten $R_5$ und $R_6$ substituiert sein kann, wobei die Reste $R_5$ und $R_6$ gleich oder verschieden sein können und die Bedeutung ($C_1$-$C_6$)-Alkyl, sowie die Bedeutung ($C_3$-$C_7$)-Cycloalkyl, ($C_1$-$C_6$)-Alkoxy, Nitro, Amino, Hydroxy, Halogen und Trifluormethyl besitzen und ferner den Ethoxycarbonylamino-Rest sowie die Gruppe Carboxyalkyloxy darstellen, bei dem die Alkylgruppe Ober 1-4 C-Atome verfügen kann, $R_1$ kann ferner ein 2-bzw. 4-Pyrimidinyl-Heterocyclus oder ein Pyridytmethyl-Rest, worin $CH_2$ in der 2-,3-, 4-Stellung stehen kann, sein, wobei der 2-Pyrimidinyl-Ring ein- oder mehrfach mit der Methylgruppe substituiert sein kann, weiterhin das mit ($C_1$-$C_6$)-Alkyl, Halogen, der Nitrogruppe, der Aminogruppe und dem ($C_1$-$C_6$)-Alkylamino-Rest substituierte 2-,3- und 4- Chinolylgerüst bedeuten, eine 2-,3- und 4-Chinolylmethylgruppe darstellen, wobei die Ringkohlenstoffe des Pyridylmethyl-und Chinolylmethyl-Restes mit ($C_1$-$C_6$)-Alkyl, ($C_1$-$C_6$)-Alkoxy, Nitro, Amino und ($C_1$-$C_6$)-Alkoxycarbonylamino substituiert sein können, $R_1$ kann weiterhin für den Fall, daß R Wasserstoff oder die Benzylgruppe bedeuten, der Säurerest einer natürlichen oder unnatürlichen Aminosäure sein, z.B. den $\alpha$-Glycyl-, den $\alpha$-Sarkosyl-, den $\alpha$-Alanyl-, den $\alpha$-Leucyl-, den $\alpha$-iso-Leucyl-, den $\alpha$-Seryl-, den $\alpha$-Phenylalanyl-, den $\alpha$-Histidyl-, den $\alpha$-Prolyl-, den $\alpha$-Arginyl-, den $\alpha$-Lysyl-, den $\alpha$-Asparagyl- und den $\alpha$-Glutamyl-Rest darstellen, wobei die Aminogruppen der jeweiligen Aminosäuren ungeschützt vorliegen oder geschützt sein können und als Schutzgruppe der Aminofunktion der Carbobenzoxy-Rest (Z-Rest) und der tert-Butoxycarbonyl-Rest (BOC-Rest) sowie die Acetylgruppe in Frage kommen und im Fall des für $R_1$ beanspruchten Asparagyl- und Glutamylrestes die zweite, nicht gebundene Carboxylgruppe als freie Carboxylgruppe oder in Form eines Esters mit $C_1$-$C_6$Alkanolen, z.B. als Methyl-, Ethyl- bzw. als tert.- Butylester vorliegt, weiterhin kann $R_1$ die Allylaminocarbonyl-2-methyl-prop-1-yl-Gruppe bedeuten. R und $R_1$ können ferner zusammen mit dem Stickstoff-Atom, an das sie gebunden sind, einen Piperazinring der Formel III oder einen Homopiperazinring bilden, sofern $R_1$ eine Aminoalkylengruppe darstellt bei dem

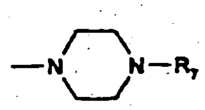

III

$R_7$ einen Alkylrest darstellt einen Phenylring bedeutet, derein-odermehrfach mit ($C_1$-$C_6$)-Alkyl, ($C_1$-$C_6$)-Alkoxy. Halogen, der Nitrogruppe, der Aminofunktion, mit ($C_1$-$C_6$)-Alkylamino, der Benzhydrylgruppe und der Bis-p-fluorbenzylhydrylgruppe substituiert sein kann.

$R_2$ die ($C_1$-$C_6$)-Alkyl-Gruppe ist, wobei die Alkylgruppe ein- oder mehrfach durch Halogen und Phenyl substituiert und das Phenyl seinerseits ein- öder mehrfach durch Halogen, ($C_1$-$C_6$)-Alkyl, ($C_3$-$C_7$)-Cycloalkyl, Carboxylgruppen, mit $C_1$-$C_6$-Alkanolen veresterten Carboxylgruppen, Trifluormethylgruppen, Hydroxylgruppen, Methoxygruppen, Ethoxygruppen oder Benzyloxygruppen substituiert sein kann, ferner kann die für $R_2$ geltende ($C_1$-$C_5$)-Alkyl-Gruppe durch die 2-Chinotylgruppe und das 2-,3- und 4-Pyridyl-Gerüst substituiert sein, die beide jeweils ein- oder mehrfach durch Halogen, ($C_1$-$C_4$)-Alkylgruppen oder ($C_1$-$C_4$)-Alkoxygruppen substituiert sein können,

$R_3$ und $R_4$ können gleich oder verschieden sein und Wasserstoff, Hydroxy, ($C_1$-$C_6$)-Alkyl, ($C_3$-$C_7$)-Cycloalkyl, ($C_1$-$C_6$)-Alkanoyl, ($C_1$-$C_6$)-Alkoxy, Halogen und Benzyloxy, weiterhin die Nitrogruppe, die Aminogruppe, die ($C_1$-$C_4$)-mono- oder dialkylsubstituierte Aminogruppe, die ($C_1$-$C_3$)-Alkoxy-carbonylamino-Funktion oder ($C_1$-$C_3$)-Alkoxycarbonylamino-($C_1$-$C_3$)-alkyl-Funktion bedeuten,

Z kann für O und S stehen,

und wobei unter der Bezeichnung Alkyl-, Alkanol-, Alkoxy- oder Alkylaminogruppe für die Reste R, $R_1$, $R_2$, $R_3$, $R_4$, $R_5$, $R_6$, $R_7$ regelmäßig sowohl "geradkettige" als auch "verzweigte" Alkylgruppen zu verstehen sind, wobei "geradkettige Alkylgruppen" beispielsweise Reste wie Methyl, Ethyl, n-Propyl, n-Butyl, n-Pentyl, n-Hexyl bedeuten können und "verzweigte Alkylgruppen" beispielsweise Reste wie Isopropyl oder tert.-Butyl bezeichnen und unter "Cycloalkyl" Reste wie beispielsweise Cyclopropyl, Cyclobutyl, Cyclopentyl, Cyclohexyl oder Cycloheptyl zu verstehen sind, außerdem die Bezeichnung "Halogen" für Fluor, Chlor, Brom oder Jod steht und die Bezeichnung "Alkoxygruppe" Reste wie beispielsweise Methoxy, Ethoxy, Propoxy, Butoxy, Isopropoxy, Isobutoxy oder Pehtoxy darstellt.

**2.** Verbindungen nach Anspruch 1,

N-(Pyridin-4-yl)-[1-(4-fluorbenzyl)-indol-3-yl]-glyoxylamid
N-(Pyridin-4-yl)-(1-methyl-indol-3-yl)- glyoxylamid
N-(Pyridin-3-yl)-[1-(4-fluorbenzyl)-indol-3-yl]-glyoxlamid
N-(Pyridin-3-yl)-(1-benzylindol-3-yl)-glyoxylamid
N-(Pyridin-3-yl)-[1-(2-chlorbenzyl)-indol-3-yl]-glyoxylamid
N-(4-Fluorphenyl)-[1-(4-fluorbenzyl)-indol-3-yl]-glyoxylamid
N-(4-Nitrophenyl)-[1-(4-fluorbenzyl)-indol-3-yl]-glyoxylamid
N-(2-Chlorpyridin-3-yl)-[1-(4-fluorbenzyl)-indoh3-yl]-glyoxylamid
N-(Pyridin-4-yl)-(1-benzylindol-3-yl)-glyoxylamid
N-(Pyridin-4-yl)-[1-(3-pyridylmethyl)-indol-3-yl]-glyoxylamid
N-(4-Fluorphenyl)-[1-(2-pyridylmethyl)-indol-3-yl]-glyoxylamid
N-(4-Fluorphenyl)-[1-(3-pyridylmethyl)-indol-3-yl]-glyoxylamid
N-(Pyridin-4-yl)-[1-(4-chlorbenzyl)-indol-3-yl]-glyoxylamid
N-(Pyridin-4-yl)-[1-(2-chlorbenzyl)-indol-3-yl]-glyoxylamid
N-(Pyridin-2-yl)-[1-(4-fluorbenzyl)-indol-3-yl]-glyoxylamid
N-(Pyridin-4-yl)-[1-(2-pyridylmethyl)-indol-3-yl]-glyoxylamid
(4-Fhenyl-piperazin-1-yl)-[1-(4-fluorbenzyl)-indol-3-yl]-glyoxylamid
N-(Pyridin-2-yl)-(1-benzyl-indol-3-yl)-glyoxylamid
4-(Pyridin-4-yl)-piperazin-1-yl)-[1-(4-flurobenzyl)-indol-3-yl]-glyoxylamid
N-(Pyndin-4-yl)-[1-(4-fluorbenzyl)-6-ethoxycarbonylamino-indol-3-yl]-glyoxylamid
N-(Pyridin-4-yl)-[1-(4-fluorbenzyl)-5-ethoxycarbonylamino-indol-3-yl]-glyoxylamid
N-(Pyridin-4-yl)-[1-(4-fiuorbenzyl)-6-cyclopentyloxycarbonylamino-indol-e-yl]-glyoxylamid
N-(3,4,5-Trimethoxybenzyl)-N-(alyaminocarbonyl-2-methyl-propl-yl)-[1-(4-fluorbenzyl)-indol-3-yl]-glyoxylamid
N-(Pyridin-4-yl)-[1-(4-fluorbenzyl)-5-methoxy-indol-3-yl]-glyoxylamid
N-(Pyridin-4-yl)-[1-(4-fluorbenzyl)-5-hydroxy-indol-3-yl]-glyoxylamid
N-(Pyridin-4-yl-[1-(4-fluorbenzyl)-5-ethoxycarbonylamino-methyl-indol-3-yl]-glyoxylamid

**3.** Verwendung der Verbindungen der Formel 1 gemäß einem der Ansprüche 1 und 2 zur Herstellung eines Arzneimittels, wobei $R_2$ in der Formel 1 auch Wasserstoff sein kann.

**4.** Verwendung der Verbindungen der Formel 1 gemäß Anspruch 1 oder 2 allein oder in Kombination untereinander zur Herstellung eines Arzneimittels mit antiasthmatiseher, antiallergischer und immunsuppressiver/immunmodulierender Wirkung für Transplantation und Krankheiten wie beispielsweise Psoriasis, rheumatoide Erkrankungen und chronische Polyarthritis, wobei $R_2$ in der Formel 1 auch Wasserstoff sein kann.

**5.** Arzneimittel, enthaltend mindestens eine Verbindung der Formel 1 nach einem der Ansprüche 1 und 2 neben üblichen Träger- und/oder Verdünnungs- beziehungsweise Hilfsstoffen, wobei $R_2$ in der Formel 1 auch Wasserstoff sein kann.

**6.** Verfahren zur Herstellung eines Arzneimittels gemäß Anspruch 5, **dadurch gekennzeichnet, daß** man eine Verbindung der Formel 1 mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form bringt.

**7.** Arzneimittel gemäß Anspruch 5 in Form von Tabletten, Dragees, Kapseln, Lösungen beziehungsweise Ampullen, Suppositorien, Pflastern, inhalativ einsetzbaren Pulverzubereitungen, Suspensionen, Cremes und Salben.

**8.** Verfahren zur Herstellung von N-substituierten Indol-3-glyoxylamiden der Formel gemäß den Ansprüchen 1 und 2, worin R, $R_1$, $R_2$, $R_3$, $R_4$ und Z die in Anspruch 1 genannte Bedeutung haben, **dadurch gekennzeichnet, daß** man

a) ein Indolderivat der Formel IV

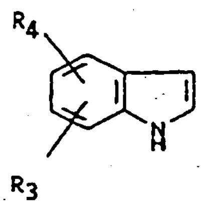

IV

worin $R_3$ und $R_4$ die genannte Bedeutung haben, in einem protischen, dipolar aprotischen oder unpolaren organischen Lösungsmittel einer suspendierten Base zufügt, mit einer reaktiven Verbindung, die den Rest $R_2$ trägt und wobei $R_2$ die genannte Bedeutung hat, umsetzt, das 1-Indol-Derivat der Formel V

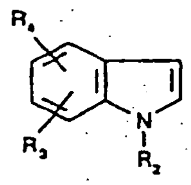

V

worin $R_2$, $R_3$ und $R_4$ die genannte Bedeutung haben, in einem aprotischen oder unpolaren organischen Lösungsmittel mit einer reaktiven Verbindung der Formel VI

$$(C(Z)\text{-}Hal)_2 \qquad VI$$

worin Z die Bedeutung Sauerstoff hat und Hal ein Halogen Fluor, Chlor, Brom oder Jod bedeutet, und danach mit einem primären oder sekundären Amin der Formel VII

$$HNRR_1 \qquad VII$$

worin R und $R_1$ die genannte Bedeutung haben, in einem aprotischen oder dipolar aprotischen Lösungsmittel umsetzt und die Zielverbindung der Formel I isoliert, oder
b) ein Indolderivat der Formel IV

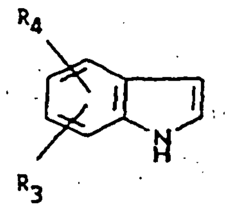

IV

worin $R_3$ und $R_4$ die genannte Bedeutung haben, in einem aprotischen oder unpolaren Lösungsmittel mit einer reaktiven Verbindung der Formel VI

$(C(Z)\text{-}Hal)_2$ VI

worin Z die Bedeutung Sauerstoff hat und Hal ein Halogen Fluor, Chlor, Brom oder Jod bedeutet, und danach in einem aprotischen oder dipolar aprotischen Lösungsmittel mit einem primären oder sekundären Amin der Formel VII

$HNRR_1$ VII

worin R und $R_1$ die genannte Bedeutung haben, umsetzt und danach das 3-Indolderivat der Formel VIII

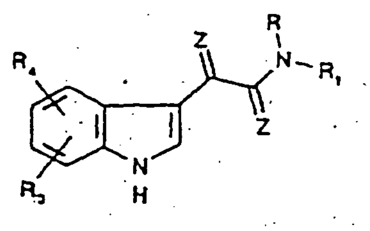

VIII

worin R, $R_1$, $R_3$, $R_4$ und Z die genannte Bedeutung haben, in einem protischen, dipolar aprotischen oder unpolaren organischen Lösungsmittel in Anwesenheit einer suspendierten Base mit einer reaktiven Verbindung, die den Rest $R_2$ trägt und wobei $R_2$ die genannte Bedeutung hat, umsetzt und die Zielverbindung der Formel I isoliert.

## Claims

**1. N-substituted indol-3-glyoxylamides of the formula 1**

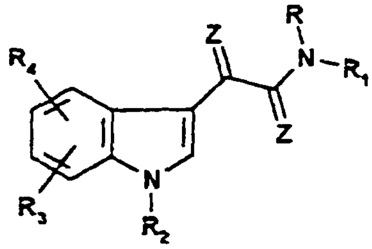

I

and their acid addition salts,
where the radicals R, $R_1$, $R_2$, $R_3$, $R_4$ and Z have the following meaning:

R = hydrogen, $(C_1\text{-}C_6)$-alkyl, where the alkyl group scan be mono- or polysubstituted by the phenyl ring. This phenyl ring, for its part, can be mono- or polysubstituted by halogen, $(C_1\text{-}C_6)$-alkyl, $(C_3\text{-}C_7)$-cycloalkyl, by carboxyl groups, carboxyl groups esterified with $(C_1\text{-}C_6)$-alkanols, trifluoromethyl groups, hydroxyl groups, methoxy

groups, ethoxy groups, benzyloxy groups and by a benyl [sic] group which is mono- or polysubstituted in the phenyl moiety by ($C_1$-$C_6$)-alkyl groups halogen atoms or trifluoromethyl groups,

$R_1$ can be a phenyl ring which is mono- or poly-substituted by ($C_1$-$C_6$)-alkyl, ($C_1$-$C_6$) -alkoxy, hydroxyl, benzyloxy, nitro, amino, ($C_2$-$C_6$)-alkylamino, ($C_1$-$C_6$)-alkoxy-carbonylamino and by a carboxyl group or a carboxyl group esterified by ($C_1$-$C_6$)-alkanols, or is a pyridin structure of the formula II

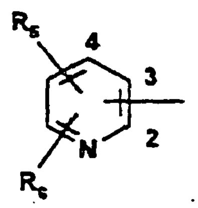

Formula II

where the pyridin structure is alternatively bonded to the ring carbon atoms 2, 3 and 4 and can be substituted by the substitutents $R_5$ and $R_6$. The radicals $R_5$ and $R_6$ can be identical or different and have the meaning ($C_1$-$C_6$)-alkyl, and also the meaning ($C_3$-$C_7$)-cycloalkyl, ($C_1$-$C_6$)-alkoxy, nitro, amino, hydroxyl, halogen and trifluoromethyl and are furthermore the ethoxycarbonylamino radical and the group carboxyalkyloxy in which the alkyl group can have 1-4 C atoms,

$R_1$ can furthermore be a 2- or 4-pyrimidinylheterocycle or a pyridylmethyl radical in which $CH_2$ can be in the 2-, 3-, 4-position where the 2-pyrimidinyl ring can be mono- or polysubstituted by the methyl group, furthermore are [sic] the 2-, 3- and 4-quinolyl structure substituted by ($C_1$-$C_6$)-alkyl, halogen, the nitro group, the amino group and the ($C_1$-$C_6$)-alkylamino radical, or are [sic] a 2-, 3- and 4-quinolyl methyl group, where the ring carbons of the pyridylmethyl and quinolylmethyl radical can be substituted by ($C_1$-$C_6$)-alkyl, ($C_1$-$C_6$)-alkoxy, nitro, amino and ($C_1$-$C_6$)-alkoxy-carbonylamino,

$R_1$ for the case where R is hydrogen or the benzyl group, can furthermore be the acid radical of a natural or unnatural amino acid, e.g. the α-glycyl, the α-sarcosyl, the α-alanyl, the α-leucyl, the α-isoleucyl, the α-seryl, the α-phenylalanyl, the α-histidyl, the α-prolyl, the α-arginyl, the α-lysyl, the α-asparagyl and the α-glutamyl radical, where the amino groups of the respective amino acids can be present in unprotected or protected form and are possible protective groups for the amino function of the carbobenzoxy radical (Z radical) and the tert-butoxycarbonyl radical (BOC radical) and also the acetyl group. In the case of the asparagyl and glutamyl radical claimed for $R_1$, the second, nonbonded carboxyl group is present as a free carboxyl group or in the form of an ester with $C_1$-$C_6$-alkanols, e.g. as the methyl, ethyl or as the tert-butyl ester. $R_1$ can furthermore be the allylaminocarbonyl-2-methylprop-l-yl group. R and $R_1$, together with the nitrogen atom to which they are bonded, can furthermore form a piperazine ring of the formula III or a homopiperazine ring if $R_1$ is an aminoalkylene group in which

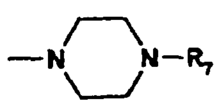

Formula III

$R_7$ is an alkyl radical, a phenyl ring which can be mono- or polysubstituted by ($C_1$-$C_6$)-alkyl, ($C_1$-$C_6$)-alkoxy, halogen, the nitro group, the amino function, by ($C_1$-$C_6$)-alkylamino, the benzhydryl group and the bis-p-fluorobenzylhydryl group,

$R_2$ is the ($C_1$-$C_6$)-alkyl group, where the alkyl group can be mono- or polysubstituted by halogen and phenyl which for its part can be mono- or polysubstituted by halogen, ($C_1$-$C_6$)-alkyl, ($C_3$-$C_7$)-cycloalkyl, carboxyl groups, carboxyl groups esterified with ($C_1$-$C_6$)-alkanols, trifluoromethyl groups, hydroxyl groups, methoxy groups, ethoxy groups or benzyloxy groups. The ($C_1$-$C_6$)-alkyl group counting as $R_2$ can furthermore be substituted by the 2-quinolyl group and the 2-, 3- and 4-pyridyl structure, which in each case can both be mono- or polysubstituted by halogen, ($C_1$-$C_4$)-alkyl groups or ($C_1$-$C_4$) - alkoxy groups,

$R_3$ and $R_4$ can be identical or different and are hydrogen, hydroxyl, ($C_1$-$C_6$)-alkyl, ($C_3$-$C_7$)-cycloalkyl, ($C_1$-$C_6$)-alkanoyl, ($C_1$-$C_6$)-alkoxy, halogen and benzyloxy. $R_3$ and $R_4$ can furthermore be the nitro group, the amino group,

the ($C_1$-$C_4$)-mono- or dialkyl-substituted amino group, and the ($C_1$-$C_3$)-alkoxycarbonylamino function or the ($C_1$-$C_3$)-alkoxy-carbonylamino- ($C_1$-$C_3$)-alkyl function,
Z is O or S,

and where the designation alkyl, alkanol, alkoxy or alkylamino group for the radicals R, $R_1$, $R_2$, $R_3$, $R_4$, $R_5$, $R_6$ and $R_7$ is normally to be understood as meaning "straight-chain" and "branched" alkyl groups, where "straight-chain alkyl groups" can be, for example, radicals such as methyl, ethyl, n-propyl, n-butyl, n-pentyl and n-hexyl and "branched alkyl groups" designate, for example, radicals such as isopropyl or tert-butyl. "Cycloalkyl" is to be understood as meaning radicals such as, for example, cyclopropyl, cyclobutyl, cyclopentyl, cyclohexyl or cycloheptyl, additionally the designation "halogen" represents fluorine, chlorine, bromine or iodine, and the designation "alkoxy group" represents radicals such as, for example, methoxy, ethoxy, propoxy, butoxy, isopropoxy, isobutoxy or pentoxy.

2. **Compounds according to Claim 1**

N-(Pyridin-4-yl)-[1-(4-fluorohenzyl)indol-3-yl]-glyoxylamide
N-(Pyridin-4-yl)-(4-methylindol-3-yl)glyoxylamide
N-(Pyridin-3-yl)-[1-(4-fluorobenzyl)-indol-3-yl]-glyoxylamide
N-(Pyridin-3-yl)-(1-benzylindol-3-yl)glyoxylamide
N-(Pyridin-3-yl)-[1-(2-chorobenzyl)indol-3-yl]-glyoxylamide
N-(4-Fluorophenyl)-[1-(4-fluorobenzyl)indol-3-yl]-glyoxylamide
N-(4-Nitrophenyl)-[1-(4-fluorobenzyl)indol-3-yl]-gloxylamide
N-(2-Chloropyridine-3-yl)-[1- (4-fluorobenzyl) indol-3-yl]glyoxylamide
N-(Pyridin-4-yl)-(-benzylindol-3-yl)glyoxylamide
N-(Pyridin-4-yl)-[1-(3-pyridylmethyl)indol-3-yl]-glyoxylamide
N-(4-Fluorophenyl)-[1-(2-pyridylmethyl)indol-3-yl]-glyoxyamide
N-(4-Fluorophenyl)-[1-(3-pyridylmethyl)indol-3-yl]-glyoxylamide
N-(Pyridin-4-yl)-[1-(4-chlorobenzyl)indol-3-yl]-glyoxylamide
N-(Pyridin-4-yl)-[1-(2-chlorobenzyl)indol-3-yl]-glyoxylamide
N-(Pyridin-2-yl)-[1-(4-fluorobenzyl)indol-3-yl]-glyoxylamide
N-(Pyridin-4-yl)-[1-(2-pyridylmethyl)indol-3-yl]-glyoxylamide
(4-Phenylpiperazin-1-yl)-[1-(4-fluorobenzyl)indol-3-yl]-glyoxylamide
N-(Pyridin-2-yl)-(1-benzylindol-3-yl)glyoxylamide
4-(Pyridin-4-yl)-piperazin-1-yl)-[1-(4-fluorobenzyl)-indol-3-yl]glyoxylamide
N-(Pyridin-4-yl)-[1-(4-fluorobenzyl)-6-ethoxycarbonylaminoindol-3-yl]glyoxylamide
N-(Pyridin-4-yl)-[1-(4-fluorobenzyl)-5-ethoxycarbonylaminoindol-3-yl]glyoxylamide
N-(Pyridin-4-)-[1-(4-fluorobenzyl)-6-cyclopentyloxycarbonylaminoindol-3-yl]glyoxylamide
N-(3,4,5-Trimethoxybenzyl)-N-(allylaminocarbonyl-2-methylprop-1-yl)-[1-(4-fluorobenzyl)indol-3-yl]-glyoxylamide
N-(Pyridin-4-yl)-[1-(4-fluorobenzyl)-5-methoxyindol-3-yl]glyoxylamide
N-(Pyridin-4-yl)-[1-(4-fluorobenzyl)-5-hydroxyindol-3-yl]glyoxylamide
N-(Pyridin-4-yl-[1-(4-fluorobenzyl)-5-ethoxycarbonylaminomethylindol-3-yl]glyoxylamide

3. Use of the compounds of the formula I according to one of claims 1 and 2 for the production of a medicament, wherein $R_2$ in the formula I can also be hydrogen.

4. Use of the compounds of the formula I according to claim 1 or 2 on their own or in combination with one another for the production of a medicament having antiasthmatic, antiallergic and immunosuppressant/immunomodulating action for transplantation and diseases such as, for example, psoriasis, rheumatoid disorders and chronic polyarthritis, wherein $R_2$ in the formula I can also be hydrogen.

5. Medicaments comprising at least one compound of the formula I according to one of claims 1 and 2 in addition to customary excipients and/or diluents or auxiliaries, wherein $R_2$ in the formula I can also be hydrogen.

6. Process for the production of a medicament according to claim 5, **characterized in that** a compound of the formula I is processed with customary pharmaceutical excipients and/or diluents or other auxiliaries to give pharmaceutical preparations or brought into a therapeutically useable form.

7. Medicaments according to claim 5 in the form of tablets, coated tablets, capsules, solutions or ampoules, suppositories, patches, powder preparations which can be employed by inhalation, suspensions, creams and ointments.

**8.** Process for the preparation of N-substituted indole-3-glyoxylamides of the formula I according to claims 1 and 2, in which R, $R_1$, $R_2$, $R_3$, $R_4$ and Z have the meaning mentioned in claim 1, **characterized in that**

a) an indole derivative of the formula IV

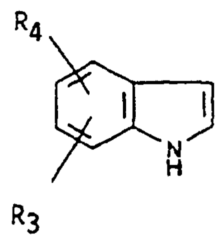

IV

in which $R_3$ and $R_4$ have the meaning mentioned, is added to a suspended base in a protic, dipolar aprotic or nonpolar organic solvent, reacted with a reactive compound which carries the radical $R_2$ and where $R_2$ has the meaning mentioned, the 1-indole derivative of the formula V

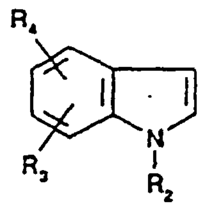

V

in which $R_2$, $R_3$ and $R_4$ have the meaning mentioned, is reacted with a reactive compound of the formula VI

$(C(Z)\text{-}Hal)_2$ VI

in which Z has the meaning oxygen and Hal is a halogen fluorine, chlorine, bromine or iodine, and then with a primary or secondary amine of the formula VII

$HNRR_1$ VII

in which R and $R_1$ have the meaning mentioned, in an aprotic or dipolar aprotic solvent and the target compound of the formula I is isolated,
or
b) an indole derivative of the formula IV

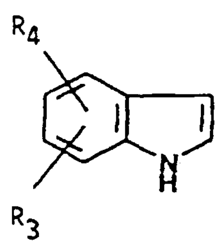

IV

in which $R_3$ and $R_4$ have the meaning mentioned, is reacted in an aprotic or nonpolar solvent with a reactive compound of the formula VI

$(C(Z)\text{-}Hal)_2$ VI

in which Z has the meaning oxygen and Hal is a halogen fluorine, chlorine, bromine or iodine, and then in an aprotic or dipolar aprotic solvent with a primary or secondary amine of the formula VII

$HNRR_1$ VII

in which R and $R_1$ have the meaning mentioned, and then the 3-indole derivative of the formula VIII

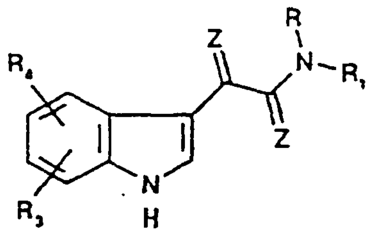

VIII

in which R, $R_1$, $R_2$, $R_3$, $R_4$ and Z have the meaning mentioned, is reacted in a protic, dipolar aprotic or nonpolar organic solvent in the presence of a suspended base with a reactive compound which carries the radical $R_2$ and where $R_2$ has the meaning mentioned, and the target compound of the formula I is isolated.

**Revendications**

**1.** Indol-3-glyoxylamides N-substitués de formule I :

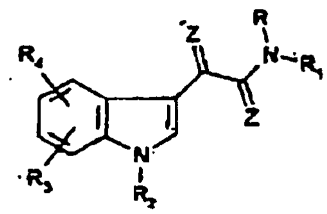

I

ainsi que leurs sels d'addition avec des acides,
dans laquelle les radicaux R, $R_1$, $R_2$, $R_3$, $R_4$ et Z ont la signification suivante :

R est hydrogène, groupement alkyle en $C_1$-$C_6$, dans lequel le groupement alkyle peut être substitué une ou plusieurs fois par le cycle phényle, ce cycle phényle pouvant de son côté être substitué une ou plusieurs fois par un halogène, par un groupement alkyle en $C_1$-$C_6$, par un groupement cycloalkyle en $C_3$-$C_7$, par des groupements carboxyle, par des groupements carboxyle estérifiés avec des alcanols en $C_1$-$C_5$, par des groupements trifluorométhyle, des groupements hydroxyle, des groupements méthoxy, des groupements éthoxy, des groupements benzyloxy ainsi que par un groupement benzyle substitué une ou plusieurs fois dans la fraction phényle par des groupements alkyle en $C_1$-$C_6$, des atomes d'halogène ou des groupements trifluorométhyle,
$R_1$ peut représenter le cycle phényle, qui est substitué une ou plusieurs fois par un groupement alkyle en $C_1$-$C_6$, alcoxy en $C_1$-$C_6$, hydroxy, benzyloxy, nitro, amino, alkyl ($C_1$-$C_6$) amino, alcoxy ($C_1$-$C_6$) carbonylamino et par le groupement carboxyle où le groupement carboxyle est estérifié par des alcanols en $C_1$-$C_6$, ou une ossature de pyridine de formule II :

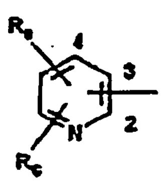

II

dans laquelle l'ossature de pyridine est éventuellement liée aux atomes de carbone 2, 3 et 4 du cycle et peut être substituée par les substituants $R_5$ et $R_6$, dans lesquels les radicaux $R_5$ et $R_6$ peuvent être identiques ou différents et représentent un groupement alkyle en $C_1$-$C_6$ ainsi qu'un groupement cycloalkyle en $C_3$-$C_7$, alcoxy en $C_1$-$C_6$, nitro, amino, hydroxy, halogène et trifluorométhyle et représentent en outre le radical éthoxycarbonylamino ainsi que le groupement carboxyalkyloxy, où le groupement alkyle peut disposer de 1 à 4 atomes de carbone,

$R_1$ peut en outre être un hétérocycle 2- ou 4-pyrimidinyle ou un radical pyridylméthyle, où $CH_2$ peut se trouver en position 2, 3, 4, dans lequel le cycle 2-pyrimidinyle peut être substitué une ou plusieurs fois par le groupement méthyle, représenter en outre l'ossature de 2-, 3-, 4-quinolyle substitué par le groupement alkyle en $C_1$-$C_6$, un halogène, le groupement nitro, le groupement amino et le radical alkyl($C_1$-$C_6$)-amino, représenter un groupement 2-, 3-, 4-quinolylméthyle, dans lequel les atomes de carbone du cycle du radical pyridylméthyle et du radical quinolylméthyle peuvent être substitués par un groupement alkyle en $C_1$-$C_6$, alcoxy en $C_1$-$C_6$, nitro, amino et alcoxy($C_1$-$C_6$)carbonylamino,

$R_1$ peut en outre, dans le cas où R représente l'hydrogène ou le groupement benzyle, être le radical acide d'un acide aminé naturel ou synthétique, par exemple les radicaux α-glycyle, α-sarkosyle, α-alanyle, α-leucyle, α-iso-leucyle, α-séryle, α-phénylalanyle, α-histidyle, α-prolyle, α-arginyle, α-lysyle, α-asparagyle et α-glutamyle, dans lesquels les groupements amino des acides aminés respectifs se présentent sous forme non protégée ou peuvent être protégés, et on citera comme groupement protecteur de la fonction amino, le radical carbobenzoxy (radical Z) et le radical tert-butoxycarbonyle (radical BOC) ainsi que le groupement acétyle ; et dans le cas des radicaux asparagyle et glutamyle revendiqués pour $R_1$, le second groupement carboxyle non lié se présente sous la forme d'un groupement carboxyle libre ou sous la forme d'un ester avec des alcanols en $C_1$-$C_6$, par exemple, sous la forme d'un ester de méthyle, d'éthyle ou de tert-butyle, $R_1$ peut en outre représenter le groupement allylaminocarbonyl-2-méthyl-prop-1-yle, R et $R_1$ peuvent en outre former conjointement avec l'atome -d'azote auquel ils sont liés, un cycle pipérazine de formule III ou un cycle homopipérazine, dans la mesure où $R_1$ représente un groupement aminoalkylène, dans lequel :

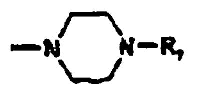

III

$R_7$ représente un radical alkyle, un cycle phényle qui peut être substitué une ou plusieurs fois par un groupement alkyle en $C_1$-$C_6$, alcoxy en $C_1$-$C_6$, halogène, le groupement nitro, la fonction amino, par le groupement alkyl ($C_1$-$C_6$)amino, le groupement benzhydryle et le groupement bis-p-fluorobenzylhydryle.

$R_2$ est le groupement alkyle en $C_1$-$C_6$, le groupement alkyle pouvant être substitué une ou plusieurs fois par un halogène et un phényle et le phényle pouvant être de son côté substitué une ou plusieurs fois par un halogène, un groupement alkyle en $C_1$-$C_6$, cycloalkyle en $C_3$-$C_7$, des groupements carboxyle, des groupements carboxyle estérifiés avec des alcanols en $C_1$-$C_6$, des groupements trifluorométhyle, des groupements hydroxyle, des groupements méthoxy, des groupements éthoxy ou des groupements benzyloxy, en outre le groupement alkyle en $C_1$-$C_6$ valant pour $R_2$ peut être substitué par le groupement 2-quinolyle et l'ossature 2-, 3- et 4-pyridyle, qui peuvent être tous deux respectivement substitués une ou plusieurs fois par un halogène, des groupements alkyle en $C_1$-$C_4$ ou des groupements alcoxy en $C_1$-$C_4$,

$R_3$ et $R_4$ peuvent être identiques ou différents et représenter l'hydrogène, les groupements hydroxy, alkyle en $C_1$-$C_6$, cycloalkyle en $C_3$-$C_7$, alcanoyle en $C_1$-$C_6$, alcoxy en $C_1$-$C_6$, halogène et benzyloxy, en outre le groupement nitro, le groupement amino, le groupement amino à substitution mono- ou dialkyle en $C_1$-$C_4$, la fonction alcoxy($C_1$-$C_3$)carbonylamino ou la fonction alcoxy($C_1$-$C_3$)carbonylamino-alkyle ($C_1$-$C_3$),

Z peut représenter O et S,

et dans lesquels on entend normalement par groupement alkyle, alcanol, alcoxy ou alkylamino pour les radicaux R, $R_1$ $R_2$ $R_3$, $R_4$, $R_5$, $R_6$, $R_7$, des groupements alkyle autant "à chaîne linéaire" que "ramifiés", "les groupements à chaîne linéaire" pouvant représenter notamment des radicaux comme les radicaux méthyle, éthyle, n-propyle, n-butyle, n-pentyle, n-hexyle et "les groupements alkyle ramifiés" désignant, par exemple, des radicaux comme l'isopropyle ou le tert-butyle et, par "cycloalkyle", on entend des radicaux comme les radicaux cyclopropyle, cyclobutyle, cyclopentyle, cyclohexyle ou cycloheptyle, en outre la désignation "halogène" représente le fluor, le chlore, le brome ou l'iode et la désignation "groupement alcoxy" représente des radicaux comme méthoxy, éthoxy, propoxy, butoxy, isopropoxy, isobutoxy ou pentoxy.

**2.** Composés selon la revendication 1,

N-(pyridin-4-yl)-1-(4-fluorobenzyl)-indol-3-yl]-glyoxylamide
N-(pyridin-4-yl)-(1-méthyl-indol-3-yl)-glyoxylamide
N-(pyridin-3-yl)-[1-(4-fluorobenzyl)-indol-3-yl]-glyoxylamide
N-(pyridin-3-yl)-(1-benzylindol-3-yl)-glyoxylamide
N-(pyridin-3-yl)-[1-(2-chlorobenzyl)-indol-3-yl]-glyoxylamide
N-(4-fluorophényl)-[1-(4-fluorobenzyl)-indol-3-yl]-glyoxylamide
N-(4-nitrophényl)-[1-(4-fluorobenzyl)-indol-3-yl)-glyoxylamide
N-(2-chloropyridin-3-yl)[1-(4-fluorobenzyl)-indol-3-yl]-glyoxylamide
N-(pyridin-4-yl)-(1-benzylindol-3-yl)-glyoxylamide
N-(pyridin-4-yl)-(3-pyridylméthyl)-indol-3-yl]-glyoxylamide
N-(4-fluorophényl)-[1-(2-pyridylméthyl)-indol-3-yl]-glyoxylamide
N-(4-fluorophényl)-[1-(3-pyridylméthyl)-indol-3-yl]-glyoxylamide
N-(pyridin-4-yl)-[1-(4-chlorobenzyl)-indol-3-yl]-glyoxylamide
N-(pyridin-4-yl)-[1-(2-chlorobenzyl)-indol-3-yl]-glyoxylamide
N-(pyridin-2-yl)-[1-(4-fluorobenzyl)-indol-3-yl]-glyoxylamide
N-(pyridin-4-yl)-[1-(2-pyridylméthyl)-indol-3-yl]-glyoxylamide
(4-phényl-pipérazin-1-yl)-[1-(4-fluorobenzyl)-indol-3-yl]-glyoxylamide
N-(pyridin-2-yl)-(1-benzyl-indol-3-yl)-glyoxylamide
4-(pyridin-4-yl)-pipérazin-1-yl)-[1-(4-fluorobenzyle)-indol-3-yl)-glyoxylamide
N-(pyridin-4-yl)-[1-(4-fluorobenzyl)-6-éthoxycarbonylamino-indol-3-yl)-glyoxylamide
N-(pyridin-4-yl)-[1-(4-fluorobenzyl)-5-éthoxycarbonylamino-indol-3-yl)-glyoxylamide
N-(pyridin-4-yl)-(1-(4-fluorobenzyl)-6-cyclopentyloxycarbonylamino-indol-3-yl-glyoxylamide
N-(3,4,5-triméthoxybenzyl)-N-(allylaminocarbonyl-2-méthyl-prop-1-yl)-[1-(4-fluorobenzyl)-indol-3-yl]-glyoxylamide
N-(pyridin-4-yl)-[1-(4-fluorobenzyl)-5-méthoxy-indol-3-yl]-glyoxylamide
N-(pyridin-4-yl)-[1-(4-fluorobenzyl)-5-hydroxy-indol-3-yl]-glyoxylamide
N-(pyridin-4-yl)-[1-(4-fluorobenzyl)-5-éthoxycarbonylaminométhyl-indol-3-yl]-glyoxylamide.

**3.** Utilisation des composés de formule I selon l'une quelconque des revendications 1 et 2 pour préparer un produit pharmaceutique, dans laquelle $R_2$ de la formule I peut aussi être l'hydrogène.

**4.** Utilisation des composés de formule I selon la revendication 1 ou 2, seuls ou en combinaison les uns aux autres, pour fabriquer un produit pharmaceutique ayant une action antiasthmatique, antiallergique et immunosuppressive/immunomodulatrice pour la transplantation et les maladies comme le psoriasis, les maladies rhumatoïdes et la polyarthrite chronique, dans laquelle $R_2$ de la formule I peut aussi être l'hydrogène.

**5.** Produit pharmaceutique contenant au moins un composé de formule I selon l'une quelconque des revendications 1 et 2 en plus des véhicules et/ou des diluants ou des adjuvants habituels, dans laquelle $R_2$ de la formule I peut aussi être l'hydrogène.

**6.** Procédé de fabrication d'un produit pharmaceutique selon la revendication 5, **caractérisé en ce que** l'on transforme un composé de formule I avec des véhicules pharmaceutiques et/ou des diluants ou d'autres adjuvants courants en préparations pharmaceutiques, en une forme applicable sur le plan thérapeutique.

**7.** Produit pharmaceutique selon la revendication 5 sous la forme de comprimés, de dragées, de capsules, de solutions ou d'ampoules, de suppositoires, de pansements, de préparations en poudre utilisables pour inhalation, de suspensions, de crèmes et de pommades.

**8.** Procédé de fabrication d'indol-3-glyoxylamides N-substitués de formule I selon les revendications 1 et 2, dans lequel R, $R_1$, $R_2$, $R_3$, $R_4$ et Z ont la signification citée dans la revendication 1, **caractérisé en ce que** l'on ajoute

a) un dérivé d'indole de formule IV :

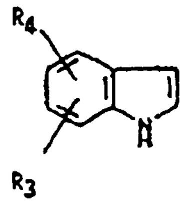

IV

dans laquelle $R_3$ et $R_4$ ont la signification citée, dans un solvant organique protique, dipolaire aprotique ou apolaire, à une base en suspension, et on le fait réagir avec un composé réactif qui porte le radical $R_2$ et dans lequel $R_2$ a la signification citée, **en ce que** l'on fait réagir le dérivé de 1-indole de formule V :

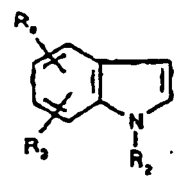

V

dans laquelle $R_2$, $R_3$ et $R_4$ ont la signification citée, dans un solvant organique aprotique ou apolaire avec un composé réactif de formule VI :

$(C(Z)\text{-}Hal)_2$ VI

dans laquelle Z représente l'oxygène et Hal représente un halogène tel que le fluor, le chlore, le brome ou l'iode et **en ce qu'**on le fait ensuite réagir avec une amine primaire ou secondaire de formule VII :

$HNRR_1$ VII

dans laquelle R et $R_1$ ont la signification citée, dans un solvant aprotique ou dipolaire aprotique et **en ce que** l'on isole le composé visé de formule I,
ou
b) **en ce qu'**on le fait réagir un dérivé d'indole de formule IV :

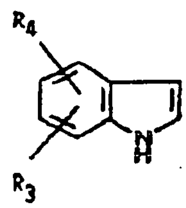

IV

dans laquelle $R_3$ et $R_4$ ont la signification citée, dans un solvant aprotique ou apolaire avec un composé réactif de formule VI :

$(C(Z)\text{-}Hal)_2$ VI

dans laquelle Z représente l'oxygène et Hal représente un halogène tel que le fluor, le chlore, le brome ou l'iode et **en ce qu'**on le fait réagir ensuite dans un solvant aprotique ou dipolaire aprotique avec une amine primaire ou secondaire de formule VII :

$HNRR_1$ VII

dans laquelle R et $R_1$ ont la signification citée, et **en ce qu'**on fait réagir ensuite le dérivé de 3-indole de formule VIII :

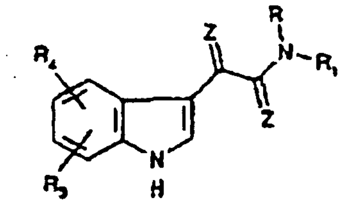

VIII

dans laquelle R, $R_1$, $R_3$, $R_4$ et Z ont la signification citée, dans un solvant organique protique, dipolaire aprotique ou apolaire, en présence d'une base en suspension avec un composé réactif qui porte le radical $R_2$, et dans laquelle $R_2$ a la signification citée, et **en ce qu'**on isole le composé visé de formule I.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- NL 6502481
- GB PS1028812 A
- EP 0675110 A1
- FR 2689888 A
- DE 1595924 A


### In der Beschreibung aufgeführte Nicht-Patentliteratur


- **G. DOMSCHKE et al.** *Ber.,* 1961, vol. 94, 2353
- *J. Med.Chem.,* 1968, vol. 11, 1252